# EUROPEAN PATENT SPECIFICATION

(11) **EP 4 051 691 B1**
(45) Date of publication and mention of the grant of the patent: **12.02.2025**
(21) Application number: 20883605.6
(22) Date of filing: 28.10.2020
(51) Int. Cl.: C07K 5/02, C07K 7/02, A61K 38/08, A61P 35/00, A61K 38/00, A61K 47/64

(54) **DOLASTATIN 10 ANALOG**
DOLASTATIN-10 ANALOG
ANALOGUE DE DOLASTATINE 10

(30) Priority: 28.10.2019 US 201962926629 P
(43) Date of publication of application: 07.09.2022
(73) Proprietor: Ariel Scientific Innovations Ltd., 4070000 Ariel (IL)
(72) Inventor: GELLERMAN, Gary, 7543696 Rishon-LeZion (IL)
(74) Representative: Nederlandsch Octrooibureau
(86) International application number: PCT/IL2020/051121
(87) International publication number: WO 2021/084532

(56) References cited:
- EP-B1- 0 598 129
- FR-A1- 2 774 989
- No further relevant documents disclosed
- YOKOSAKA S ET AL.: "Synthesis and evaluation of novel dolastatin 10 derivatives for versatile conjugations", BIOORGANIC & MEDICINAL CHEMISTRY, vol. 26, no. 8, 1 May 2018 (2018-05-01), pages 1643 - 1652, XP055635481, DOI: 10.1016/j.bmc.2018.02.011

## Description

### RELATED APPLICATIONS

This application claims the benefit of priority of U.S. Provisional Patent Application No. 62/926,629 filed on 28 October 2019.

### FIELD AND BACKGROUND OF THE INVENTION

The present invention, in some embodiments thereof, relates to a dolastatin 10 analog, and more particularly, but not exclusively, to synthesis and uses of compounds derived from the dolastatin 10 analog and conjugates comprising the same.

Dolastatin 10 is a marine natural product isolated from the Indian Ocean sea hare Dollabella auricularia and from the marine cyanobacterium Symploca sp. VP642 from Palau. Being a small linear peptide molecule, dolastatin 10 is considered a promising anti-cancer drug showing potency against breast and liver cancers, solid tumors and some types of leukemias. Preclinical research indicated potency in experimental antineoplastic and tubulin assembly systems.

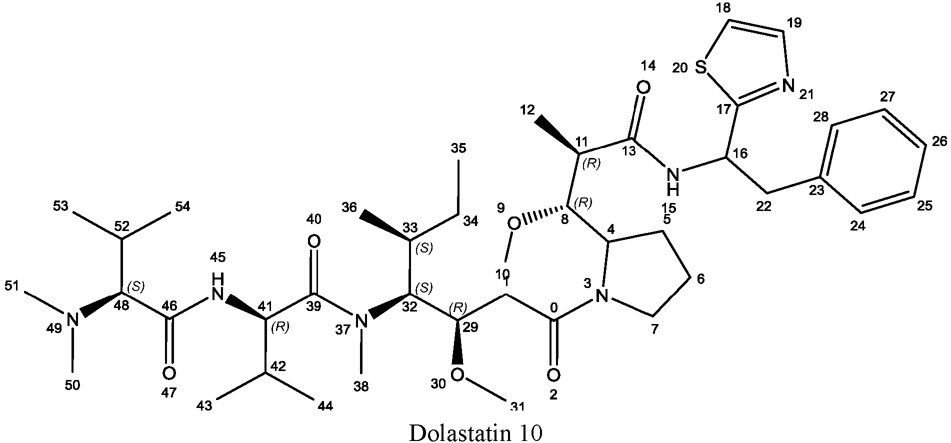

Dolastatin 10 is a mitotic inhibitor that acts on the microtubule assembly by interfering with tubulin formation and thereby disrupt cell division by mitosis and induces apoptosis and Bcl-2 phosphorylation in several malignant cell types. It also noncompetitively inhibits binding of vincristine to tubulin (at a location known as the vinca/peptide region), while having been shown to bind to the RZX/MAY region.

Dolastatin 10 is chemically related to symplostatin and analogues thereof, and the family of auristatins are potent synthetic analogs of dolastatin 10. Dolastatin 10 has been shown to inhibit the growth of L1210 murine leukemia cells in culture, with a concordant rise in the mitotic index, and its IC50 value for cell growth was 0.5 nM. Comparable values for the other drugs were 0.5 nM for maytansine, 1 nM for rhizoxin, 20 nM for vinblastine, and 7 µM for phomopsin A. IC50 values were also obtained for the polymerization of purified tubulin in glutamate: 1.2 µM for dolastatin 10, 1.4 µM for phomopsin A, 1.5 µM for vinblastine, 3.5 µM for maytansine, and 6.8 µM for rhizoxin. Dolastatin 10 and vinblastine were comparable in their effects on microtubule assembly dependent on microtubule associated proteins. Preliminary studies indicated that dolastatin 10, like vinblastine, causes formation of a cold-stable tubulin aggregate at higher drug concentrations. Dolastatin 10 was shown to be the strongest inhibitor of the binding of radiolabeled vinblastine and vincristine to tubulin.

Bai, Ruoli, et al. ["Differential effects of active isomers, segments, and analogs of dolastatin 10 on ligand interactions with tubulin: correlation with cytotoxicity", Biochemical pharmacology, 1993, 45(7), pp. 1503-1515] disclose the preparation of six chiral isomers, one tri-and one tetrapeptide segment, and one pentapeptide analog of dolastatin 10, all of which differ little from dolastatin 10 as inhibitors of tubulin polymerization, in order to examine the mechanism of action of dolastatin 10.

Miyazaki, Koichi, et al. ["Synthesis and antitumor activity of novel dolastatin 10 analogs", Chemical and pharmaceutical bulletin, 1995, 43(10), pp. 1706-1718], disclose the synthesis and antitumor activity evaluation of a family of dolastatin 10 analogs, each modified at one of the constituent amino acid derivatives.

Pettit, R.K. et al. ["Specific activities of dolastatin 10 and peptide derivatives against Cryptococcus neoformans., Antimicrobial agents and chemotherapy, 1998, 42(11), pp. 2961-2965], disclose the evaluation of the antifungal spectrum of dolastatin 10 and four structural modifications thereof.

Maderna, Andreas, et al. ["Discovery of cytotoxic dolastatin 10 analogues with N-terminal modifications", Journal of medicinal chemistry, 2014, 57(24), pp. 10527-10543], disclose auristatins as synthetic analogues of the antineoplastic natural product Dolastatin 10, and reports their ultrapotent cytotoxic microtubule inhibitory activity and their use as payloads in antibody-drug conjugates (ADCs).

European patent EP 0 598 129, French patent application FR 2 774 989 A1 and Shinya Y. et al. ["Synthesis and evaluation of novel dolastatin 10 derivatives for versatile conjugations", Bioorganic & Medicinal Chemistry, 2018, 26(8), pp. 1643-1652] disclose the design and synthesis of a series of dolastatin 10 analogs useful as payloads for conjugated drugs.

U.S. Patent Application Publication Nos. 20160068566, 20160083420, 20160068567, 20180141973, 20200010414, 20180222858, and U.S. Patent No. 10,351,593, disclose various aspects of derivatives and analogs of dolastatin 10 or auristatins.

Additional prior art documents include WO/2003/008378, WO/2014/174062, WO/2014/174064, WO/2014/174060, WO/2016/192527 and WO/2006/063707, WO/2006/063707.

### SUMMARY OF THE INVENTION

The present invention, in some embodiments thereof, relates to a dolastatin 10 analog, and more particularly, but not exclusively, to synthesis and uses of compounds derived from the dolastatin 10 analog and conjugates comprising the same.

Thus, according to an aspect of some embodiments of the present invention there is provided a dolastatin 10 analog have general Formula I: 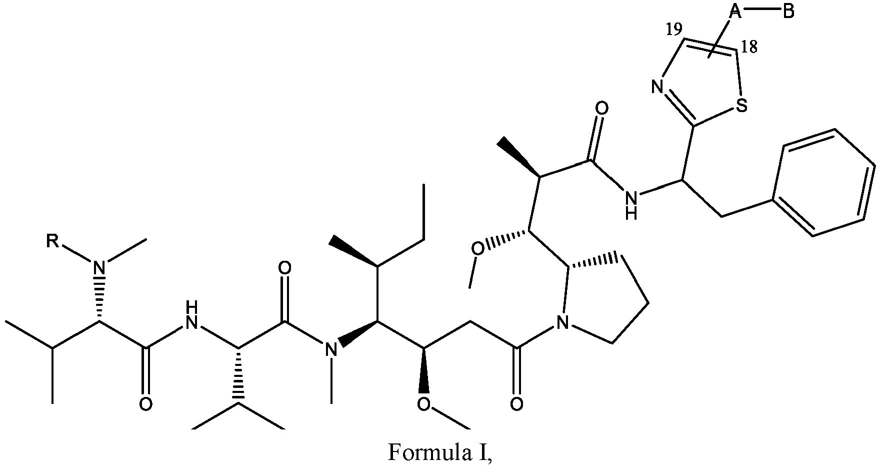 wherein:
A is a linear or branched C₁₋₆ alkyl attached to a thiazole group at position 18 or 19;
B is a functional group; and
R is an alkyl or H.

In some embodiments, the linear or branched C₁₋₆ alkyl is selected from the group consisting of -CH₂-, -CHCH₃-, -C(CH₃)₂-, -(CH₂)₂-, -CH₂CHCH₃-, and -CH₂C(CH₃)₂-.

In some embodiments, the linear or branched C₁₋₆ alkyl is attached at position 18 on the thiazole group.

In some embodiments, the linear or branched C₁₋₆ alkyl is a methylene group (-CH₂-).

In some embodiments, the functional group is selected from the group consisting of an alkenyl, an alkynyl, a halo, a hydroxyl, a carbonyl, an aldehyde, a haloformyl (acyl halide), a carbonate ester, a carboxylate, a carboxyl, a carboalkoxy (ester), an alkoxy, a hydroperoxy, a peroxy, an ether, a hemiacetal, a hemiketal, an acetal, a ketal, an orthoester, a methylenedioxy, an orthocarbonate ester, a carboxylic anhydride, a carboxamide, an a amine, a ketamine, an aldimine, an imide, an azide, an azo (diimide), a cyanate, an isocyanate, a nitrate, a nitrile, an isonitrile, a nitrosooxy, a nitro, a nitroso, an oxime, a pyridyl, a carbamate, a sulfhydryl, a sulfide, a disulfide, a sulfinyl, a sulfonyl, a sulfino, a sulfo, a sulfo-ester, a thiocyanate, an isothiocyanate, a carbonothioyl, a carbonothioyl, a carbothioic S-acid, a carbothioic O-acid, a thiolester, a thionoester, a carbodithioic acid, a carbodithio, a phosphino, a phosphono, a phosphate, and a phosphate.

In some embodiments, the functional group is a hydroxyl.

In some embodiments, R is methyl.

In some embodiments, A is -(CH₂)- attached at position 18, B is -OH, and the dolastatin 10 analog is having a structure:

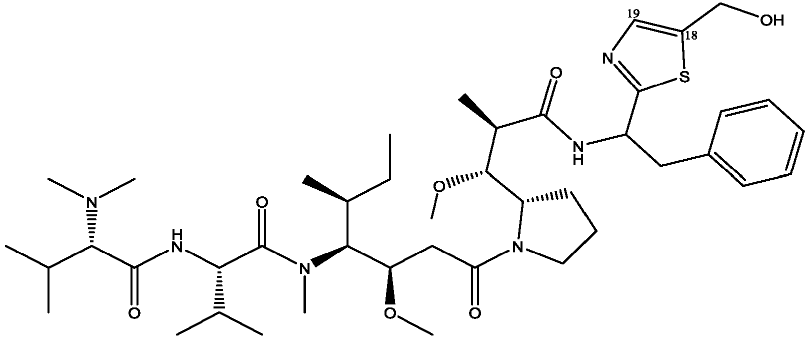

### (2S)-2-((S)-2-(dimethylamino)-3-methylbutanamido)-N-((3R,4S,5S)-1-((2S)-2-((1R,2R)-3-((1-(5-(hydroxymethyl)thiazol-2-yl)-2-phenylethyl)amino)-1-methoxy-2-methyl-3-oxopropyl)pyrrolidin-1-yl)-3-methoxy-5-methyl-1-oxoheptan-4-yl)-N,3-dimethylbutanamide.

According to another aspect of some embodiments of the present invention, there is provided a conjugate, which includes:
a moiety of a dolastatin 10 analog according to as provided herein 1-8,
a functional moiety, and
a linking moiety connecting the dolastatin 10 analog moiety and the functional moiety.

In some embodiments, the linking moiety comprises a biocleavable bond or group selected from the group consisting of an amide, an ester, a carbamate, a carbonate, a disulfide, a sulfonamide, an ether, a thioether, a valine-citrulline, a hydrazine and an oxyacrylate.

In some embodiments, the linking moiety comprises a disulfide.

In some embodiments, the functional moiety is a bioactive agent, a labeling agent and/or a diagnostic agent.

In some embodiments, the functional moiety is selected from the group consisting of a peptide, a protein, an antibody, a biodegradable polymer, a targeting agent, a drug, a dye, a nanoparticle, a bead, a photodynamic therapy sensitizer, radiotherapy agent, a metal complex, an anti-cancer agent, an anti-proliferative agents, chemosensitizing agents, an anti-inflammatory agent, an antimicrobial agent, an anti-oxidant, a hormone, an anti-hypertensive agent, an anti-diabetic agent, an immunosuppressant, an enzyme inhibitor, a neurotoxin and an opioid.

In some embodiments, the functional moiety is a somatostatin analog.

In some embodiments, the dolastatin 10 analog comprises a -CH₂-OH at its thiazole, the functional moiety is a octreotide moiety, and the linking moiety comprises a disulfide.

In some embodiments, the conjugate provided herein is selected from the group consisting of conjugate **8b,** conjugate **8c,** or conjugate **8d.**

According to another aspect of some embodiments of the present invention, there is provided a process of preparing the dolastatin 10 analog provided herein, which includes:
attaching an amine-protected derivative of (2-(1-(λ²-azaneyl)-2-phenylethyl)thiazol-5-yl)methanol (**Bu-5**) to a 2-chlorotrityl chloride solid-support resin to thereby form a nascent peptide,
coupling an amine-protected derivative of (2R,3R)-3-methoxy-2-methyl-3-((S)-pyrrolidin-2-yl)propanoic acid (**Bu-1**) to the nascent peptide on the resin,
coupling an amine-protected derivative of (3R,4S,5S)-3-methoxy-5-methyl-4-(methylamino)heptanoic acid (**Bu-2**) to the nascent peptide on the resin,
coupling an amine-protected derivative of Val-OH to the nascent peptide on the resin,
coupling dimethyl-Val to the nascent peptide on the resin, and
cleaving the dolastatin 10 analog from the resin to thereby obtain (2S)-2-((S)-2-(dimethylamino)-3-methylbutanamido)-N-((3*R*,4*S*,5*S*)-1-((2*S*)-2-((1*R*,2*R*)-3-((1-(5-(hydroxymethyl)thiazol-2-yl)-2-phenylethyl)amino)-1-methoxy-2-methyl-3-oxopropyl)pyrrolidin-1-yl)-3-methoxy-5-methyl-1-oxoheptan-4-yl)-N,3-dimethylbutanamide.

According to another aspect of some embodiments of the present invention, there is provided a process of preparing the conjugate as provided herein, which includes coupling the dolastatin 10 analog of as provided herein 1-8 to the functional moiety, thereby forming the linking moiety.

According to another aspect of some embodiments of the present invention, there is provided a process of preparing the conjugate as provided herein, which includes:
forming the functional moiety on a solid-support resin using solid-state peptide synthesis protocols, wherein the functional moiety comprises a linkable group,
forming the dolastatin 10 analog starting from the linkable group, thereby forming the linking moiety on a solid-support resin using solid-state peptide synthesis protocols, and
releasing the conjugate from the resin.

According to another aspect of some embodiments of the present invention, there is provided a pharmaceutical composition that includes the conjugate as provided herein, and a pharmaceutically acceptable carrier.

According to another aspect of some embodiments of the present invention, there are provided compounds for use in a method of treating a medical condition treatable with dolastatin 10 in a subject, which includes administering to the subject a therapeutically effective amount of the conjugate as provided herein.

According to another aspect of some embodiments of the present invention, there is provided a use of the conjugate as provided herein in the preparation of a medicament useful in the treatment of a medical condition treatable with dolastatin 10 in a subject.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### DESCRIPTION OF SOME SPECIFIC EMBODIMENTS OF THE INVENTION

The present invention, in some embodiments thereof, relates to a dolastatin 10 analog, and more particularly, but not exclusively, to synthesis and uses of compounds derived from the dolastatin 10 analog.

Before explaining at least one embodiment of the invention in detail, it is to be understood that the invention is not necessarily limited in its application to the details set forth in the following description or exemplified by the Examples. The invention is capable of other embodiments or of being practiced or carried out in various ways.

As presented hereinabove, dolastatin 10 is a potent antimitotic peptide isolated from the marine mollusk Dolabella auricularia. Four of its five residues are modified amino acids (in sequence, dolavaline, valine, dolaisoleuine, dolaproine, dolaphenine). Besides inhibiting tubulin polymerization, dolastatin 10 noncompetitively inhibits vinca alkaloid binding to tubulin, inhibits nucleotide exchange and formation of the βs cross-link, and stabilizes the colchicine binding activity of tubulin.

It is difficult to link dolastatin 10 to potential carriers due to lack of functional groups suitable for effective conjugation. Therefore, modified analogs known in the art are used for conjugation through modified N-terminus (typically, monomethyl Val or alpha-amino isobutyric acid).

While conceiving the present invention, the inventor contemplated a dolastatin 10 analog that would allow the potent peptide to be conjugated to a bioactive, diagnostic or labeling moiety, such as an antibody, a nanoparticle, a protein, a biodegradable polymer, a peptide, and the likes, with minimal reduction of activity compared to the activity of the naturally occurring molecule. The inventor had further contemplated a modification that introduces a functional group to dolastatin 10 - one which would allow conjugating a dolastatin 10 analog to a bioactive, diagnostic or labeling moiety via a biocleavable linker. The inventor had further contemplated a modification that would be conducive to solid-state peptide synthesis methodologies that would open the possibility of rapid, effective and versatile high-throughput preparation and screening of series of analogs and conjugates thereof.

While reducing the present invention to practice, the inventor directed the abovementioned modification to the C-terminus of dolastatin 10, particularly to the thiazole moiety that exhibits two aromatic carbon positions capable of various substitutions (positions 18 and 19 in the structure presented in the Background section above). The inventor has further contemplated that for the modification to have minimal effect of the biological activity of the analog, the substitution at the abovementioned positions should have minimal effect of the electronic configuration of the molecule, particularly the π-conjugated system of the thiazole moiety - hence, the modification should have an aliphatic carbon separating the analog's π-conjugated system from the functional group.

As a proof of concept the inventor synthesized a dolastatin 10 analog referred to herein as "Dolas-Ol", which differs from the natural substance by the hydroxymethylene (CH₂OH) tether at thiazole ring. Such modification preserves N-terminus (dimethyl Val) and in the same time enables conjugation to a bioactive, diagnostic or labeling moiety through the CH₂OH moiety at the C-terminus.

### Dolastatin 10 analog:

Thus, according to an aspect of some embodiments of the present invention, there is provided a dolastatin 10 analog, represented by the general Formula I: 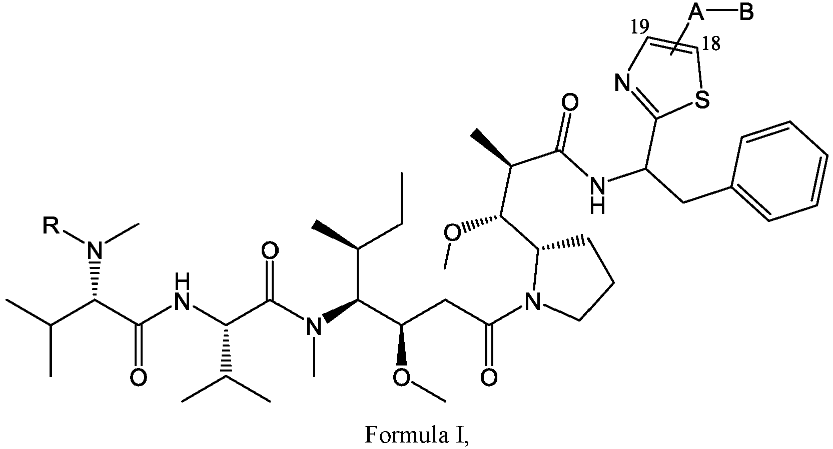 wherein:
A is a linear or branched C₁₋₆ alkyl attached to a thiazole group at position 18 or 19;
B is a functional group; and
R is an alkyl or H.

As used herein, the words "link", "linked", "linkage" "linker", "bound", "coupled" or "attached", are used interchangeably herein and refer to the presence of at least one covalent bond between species and moieties, unless specifically noted otherwise. The phrase "covalent bond", as used herein, refers to one or more pairs of electrons that are shared between atoms in a form of chemical bonding.

The term "analog" as used herein may refer to a compound in which one or more atoms are replaced with a different atom or group of atoms. The term may also refer to compounds with an identity of atoms but of different isomeric configuration. Such isomers may be constitutional isomers, i.e. structural isomers having different bonding arrangements of their atoms or stereoisomers having identical bonding arrangements but different spatial arrangements of the constituent atoms. The term may also refer to a biological activity of the related compounds (analogous to each other), which may differ in intensity, or reactivity of the compounds in some aspects, but resemble each other in the essence of the biological activity, as in the case of the naturally occurring dolastatin 10 and Dolas-Ol, presented herein.

The dolastatin 10 analog presented herein also exhibits at least one aliphatic carbon separating the thiazole's π-conjugated system from the functional group, referred to herein as a linear or branched C₁₋₆ alkyl. In some embodiments the linear or branched C₁₋₆ alkyl is selected from the group consisting of -CH₂-, -CHCH₃-, -C(CH₃)₂-, -(CH₂)₂-,-(CH₂)₃-, -(CH₂)₄-, -CH₂CHCH₃-, -(CH₂)₅-, -(CH₂)₆-, -(CH(CH₃))-CH₂- and -CH₂C(CH₃)₂-. In some embodiments, the linear or branched C₁₋₆ alkyl is attached at position 18 on the thiazole group of the dolastatin 10 analog. In some embodiments, the linear or branched C₁₋₆ alkyl is a methylene group (-CH₂-).

According to some embodiments of the present invention, the aliphatic carbon may further be extended by a spacer moiety, such as, without limitation, -CH₂-O-, -(CH₂)₂-O-, -(CH₂)₃-O-, - CH=CH-CH=CH-, -C≡C-C≡C-, -CH₂CH(OH)CH₂-, -CH₂-O-CH₂-, -CH₂-O-CH₂-O-, -(CH₂)₂-O-(CH₂)₂-, -(CH₂)₂-O-(CH₂)₂-O-, -CH₂-*m*C₆H₄-CH₂-, -CH₂-*m*C₆H₄-CH₂-O-, -CH₂-*p*C₆H₄-CH₂-, - CH₂-*p*C₆H₄-CH₂-O-, -CH₂-NHCO-, -C₆H₄-NHCO-, -CH₂-O-CH₂- and -CH=CH-CH₂-NH-(CH₂)₂-.

The dolastatin 10 analog presented herein exhibits a functional group attached on the thiazole moiety thereof via an alkyl group. The term "functional group" as used herein, refers to an atom or a group of atoms that imparts a particular chemical function to a molecule bearing the same, such as amines, carboxylates, and the like. The phrase "reactive group", which is used interchangeably with the term "functional group", refers to a chemical group that is capable of undergoing a chemical reaction that typically leads to the formation a covalent bond. Chemical reactions that lead to a bond formation include, for example, cycloaddition reactions (such as the Diels-Alder's reaction, the 1,3-dipolar cycloaddition Huisgen reaction, and the similar "click reaction"), condensations, nucleophilic and electrophilic addition reactions, nucleophilic and electrophilic substitutions, addition and elimination reactions, alkylation reactions, rearrangement reactions and any other known organic reactions that involve a reactive group.

Exemplary functional/reactive groups, contemplated as the context of embodiments of the present invention (e.g., Formula I) include, without limitation, an alkenyl, an alkynyl, a halo, a hydroxyl, a carbonyl, an aldehyde, a haloformyl (acyl halide), a carbonate ester, a carboxylate, a carboxyl, a carboalkoxy (ester), an alkoxy, a hydroperoxy, a peroxy, an ether, a hemiacetal, a hemiketal, an acetal, a ketal, an orthoester, a methylenedioxy, an orthocarbonate ester, a carboxylic anhydride, a carboxamide, an a amine, a ketamine, an aldimine, an imide, an azide, an azo (diimide), a cyanate, an isocyanate, a nitrate, a nitrile, an isonitrile, a nitrosooxy, a nitro, a nitroso, an oxime, a pyridyl, a carbamate, a sulfhydryl, a sulfide, a disulfide, a sulfinyl, a sulfonyl, a sulfino, a sulfo, a sulfo-ester, a thiocyanate, an isothiocyanate, a carbonothioyl, a carbonothioyl, a carbothioic S-acid, a carbothioic O-acid, a thiolester, a thionoester, a carbodithioic acid, a carbodithio, a phosphino, a phosphono, a phosphate, and a phosphate, as these terms are known and widely used in the art.

In some embodiments, the functional group is a hydroxyl.

In some embodiments, the R in Formula I is methyl.

In some embodiments, A is -(CH₂)- attached at position 18, B is -OH, and the dolastatin 10 analog, also referred to herein as "Dolas-Ol", is having a structure:

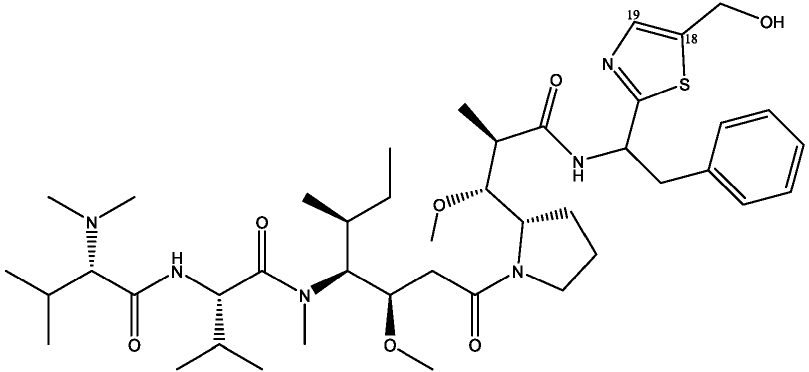

### (2S)-2-((S)-2-(dimethylamino)-3-methylbutanamido)-N-((3R,4S,5S)-1-((2S)-2-((1R,2R)-3-((1-(5-(hydroxymethyl)thiazol-2-yl)-2-phenylethyl)amino)-1-methoxy-2-methyl-3-oxopropyl)pyrrolidin-1-yl)-3-methoxy-5-methyl-1-oxoheptan-4-yl)-N,3-dimethylbutanamide

According to an aspect of some embodiments of the present invention, there is provided a compound referred to herein as "Dolas-Ol", and having the IUPAC name (2S)-2-((S)-2-(dimethylamino)-3-methylbutanamido)-N-((3R,4S,5 S)-1-((2S)-2-((1R,2R)-3-((1-(5-(hydroxymethyl)thiazol-2-yl)-2-phenylethyl)amino)-1-methoxy-2-methyl-3-oxopropyl)pyrrolidin-1-yl)-3-methoxy-5-methyl-1-oxoheptan-4-yl)-N,3-dimethylbutanamide.

### Conjugates of dolastatin 10 analog:

The dolastatin 10 analog provided herein can be used as a stand-alone bioactive agent, or be tethered to a functional agent to form a conjugate; such conjugate is designed to exert activity by both sides thereof, namely the activity exerted by the dolastatin 10 analog and the activity exerted by the functional agent. The conjugate can be designed to stay as a conjugate (intact), or disassociate (cleave) into at least the two parts comprising the same.

Thus, according to yet another aspect of some embodiments of the present invention, there is provided a conjugate of dolastatin 10 analog, which includes:
a moiety of a dolastatin 10 analog, according to some embodiments of the present invention,
a functional moiety, and
a linking moiety connecting the dolastatin 10 analog moiety via the functional group denoted in Formula I, and the functional moiety.

In the context of the present disclosure, the term "moiety" is used to denote a portion of a molecule, which may be a functional group, or describe a portion of a molecule with multiple functional groups which share common structural aspects. In some embodiments, the term "moiety" refers to the major part of a molecule being attached to another molecule or a functional group. In some embodiments, the moiety substantially exhibits the same biologic activity of the molecule. In some embodiments, the moiety can generate the molecule upon cleavage from a conjugate comprising the same. In the context of embodiments of the present invention, the term "moiety" is used similarly to the term "residue" in the context of amino acids in a polypeptide.

### Structural elements in the conjugate:

As used herein, the term "linking moiety" describes a chemical moiety (a group of atoms or a covalent bond) that links two chemical moieties via one or more covalent bonds. A linking moiety may include atoms that form a part of one or both of the chemical moieties it links, and/or include atoms that do not form a part of one or both of the chemical moieties it links. For example, a peptide bond (amide) linking moiety that links two amino acids includes at least a nitrogen atom and a hydrogen atom from one amino acid and at least a carboxyl of the other amino acid. In general, the linking moiety can be formed during a chemical reaction, such that by reacting two or more functional/reactive groups, the linking moiety is formed as a new chemical entity which can comprise a bond (between two atoms), or one or more bonded atoms. Alternatively, the linking moiety can be a chemical moiety comprising two or more reactive groups to which the reactive groups of other compounds can be attached, either directly or indirectly.

In the context of some embodiments of the present invention, the term "linking moiety" is synonymous with the term "cleavable linker", meaning that the linking moiety is selected or designed to break under certain conditions, or at certain locations in the treated subject. The term "biocleavable linker", for example, refers to a cleavable linker that, *inter alia,* breaks under physiological conditions.

The positions at which the elements of the conjugate are linked to one-another are generally selected such that once cleaved off the conjugate, any or at least some of the moieties stemming from the cleavage substantially exhibit their original biological activity (mechanism of biological activity), or at least some thereof. According to some embodiments of the present invention, the linking moiety is formed such that the biological activity of the elements of the conjugate remains substantially the same as the biological activity of the standalone elements, while still in the conjugated form. In some embodiments, the functional moiety of the conjugate can be regarded as a prodrug as long as it is bound to the conjugate that regains its original functional activity once cleaved-off the conjugate.

Representative examples of reactive (functional) groups that may be used to form a cleavable linker include, without limitation, acyl halide, aldehyde, alkoxy, alkyne, amide, amine, aryloxy, azide, aziridine, azo, carbamate, carbonyl, carboxyl, carboxylate, cyano, diene, dienophile, epoxy, guanidine, guanyl, halide, hydrazide, hydrazine, hydroxy, hydroxylamine, imino, isocyanate, isothiocyanate, maleimide, N-hydroxycuccinimide, carboxylic acid halide, alkyl halide, nitro, phosphate, phosphonate, sulfinyl, sulfonamide, sulfonate, thioalkoxy, thioaryloxy, thiocarbamate, thiocarbonyl, thiohydroxy, thiourea and urea, as these terms are defined hereinafter.

According some embodiments of the present invention, various elements of the conjugate presented herein are attached to one or more linking moieties via spacer moieties. As used herein, the phrase "spacer moiety" describes a chemical moiety that typically extends between two chemical moieties and is attached to each of the chemical moieties via covalent bonds. The spacer moiety may be linear or cyclic, be branched or unbranched, rigid or flexible, hydrophobic or hydrophilic.

The nature of the spacer moieties can be regarded as having an effect on two aspects, the synthetic aspect, namely the influence of the spacer moieties on the process of preparing the conjugates presented herein, and the influence of the spacer moieties on the biology activity of the conjugates in terms of drug-release profile(s), biological activity, bioavailability and other ADME-Tox considerations.

According to some embodiments of the present invention, the spacer moieties are selected such that they allow and/or promote the conjugation reaction between various elements of the conjugates presented herein, and reduce the probability for the formation of side-products due to undesired reactions. Such traits can be selected for in terms of spacer's length, flexibility, structure and specific chemical reactivity or lack thereof. Spacer moieties with fewer reactive groups will present a simpler synthetic challenge, requiring less protection/deprotection steps and affording higher chemical yields. For example, saturated and linear alkyls of 1-10, or 1-5 carbon atoms, having one reactive group at the end atom for conjugation with a corresponding reactive group, would afford substantially higher yield and fewer side products. Similarly, a spacer moiety based on one or two chained benzyl rings would also lead to an efficient conjugation reaction.

According to some embodiments of the present invention, the spacer moieties are selected such that they provide favorable cleavage conditions, as these are discussed hereinbelow. For example, a spacer may alter the accessibility of an enzyme to the linking moiety, thereby allowing the enzyme to cleave the linkage between the bioactive agent and the conjugate. In some embodiments, a spacer moiety can be regarded as forming a part of a linking moiety.

According to some embodiments of the present invention, the spacer moieties include, without limitation, -CH₂-, -CH₂-O-, -(CH₂)₂-, -(CH₂)₂-O-, -(CH₂)₃-, -(CH₂)₃-O-, -(CH₂)₄-, -(CH₂)₅-, -(CH₂)₆-, -(CH(CH₃))-CH₂-, -CH=CH-CH=CH-, -C≡C-C≡C-, -CH₂CH(OH)CH₂-, -CH₂-O-CH₂-, -CH₂-O-CH₂-O-, -(CH₂)₂-O-(CH₂)₂-, -(CH₂)₂-O-(CH₂)₂-O-, -CH₂-*m*C₆H₄-CH₂-, -CH₂-*m*C₆H₄-CH₂-O-, -CH₂-*p*C₆H₄-CH₂-, -CH₂-*p*C₆H₄-CH₂-O-, -CH₂-NHCO-, -C₆H₄-NHCO-, -CH₂-O-CH₂- and -CH=CH-CH₂-NH-(CH₂)₂-.

Examples of linking moieties, according to some embodiments of the present invention, include without limitation, acetal, aldimine, amide, aminal, aminoacetal, carbamate, carbonate, carboxylate, cycloalkene, cyclohexene, disulfide, ester, heteroalicyclic, heteroaryl, hydrazone, imide, imine, ketal, ketimine, lactam, lactone, oxime, phosphate ester, semicarbazone, thioacetal, thioketal, triazine, triazole, and the like. Other linking moieties are defined hereinbelow, and further other linking moieties are contemplated within the scope of the term as used herein.

According to some embodiments, the cleavable linker, or labile linking moiety, is selected from the group consisting of an amide, an ester, a carbamate, a carbonate, a disulfide, a sulfonamide, an ether, a thioether, a valine-citrulline, a hydrazine and an oxyacrylate. In some preferred embodiments, the linking moiety comprises a disulfide.

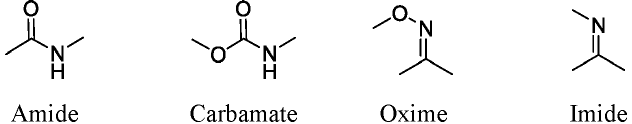

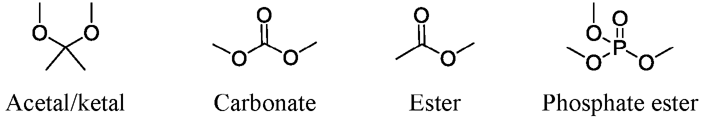

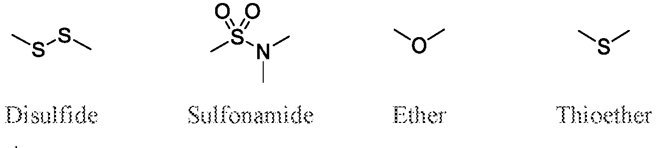

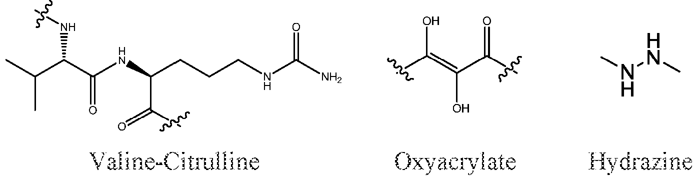

Definitions of specific functional groups, chemical terms, and general terms used throughout the specification are described in more detail below. For purposes of this invention, the chemical elements are identified in accordance with the Periodic Table of the Elements, CAS version, Handbook of Chemistry and Physics, 75th Ed., inside cover, and specific functional groups are generally defined as described therein. Additionally, general principles of organic chemistry, as well as specific functional moieties and reactivity, are described in Organic Chemistry, Thomas Sorrell, University Science Books, Sausalito, 1999; Smith and March March's Advanced Organic Chemistry, 5th Edition, John Wiley & Sons, Inc., New York, 2001; Larock, Comprehensive Organic Transformations, VCH Publishers, Inc., New York, 1989; Carruthers, Some Modern Methods of Organic Synthesis, 3rd Edition, Cambridge University Press, Cambridge, 1987.

As used herein, the terms "amine" or "amino", describe both a -NR'R" end group and a -NR'- linking moiety, wherein R' and R" are each independently hydrogen, alkyl, cycloalkyl, aryl, as these terms are defined hereinbelow.

Herein throughout, the phrase "end group" describes a chemical group that is attached to one compound (a substituent; a reactive group; a functional group etc.), while the term "linking moiety" describes a group that is attached to two compounds and links therebetween.

The amine group can therefore be a primary amine, where both R' and R" are hydrogen, a secondary amine, where R' is hydrogen and R" is alkyl, cycloalkyl or aryl, or a tertiary amine, where each of R' and R" is independently alkyl, cycloalkyl or aryl.

Alternatively, R' and R" can each independently be hydrogen, hydroxyalkyl, trihaloalkyl, cycloalkyl, alkenyl, alkynyl, aryl, heteroaryl, heteroalicyclic, amine, halo, sulfonate, sulfoxide, phosphonate, hydroxy, alkoxy, aryloxy, thiohydroxy, thioalkoxy, thioaryloxy, cyano, nitro, azo, azido, sulfonamide, carbonyl, C-carboxylate, O-carboxylate, N-thiocarbamate, O-thiocarbamate, urea, thiourea, N-carbamate, O-carbamate, C-amide, N-amide, guanyl, guanidine and hydrazine, as these terms are defined herein.

The term "alkyl" describes a saturated aliphatic hydrocarbon including straight chain (unbranched) and branched chain groups. Preferably, the alkyl group has 1 to 20 carbon atoms. Whenever a numerical range; e.g., "1-20", is stated herein, it implies that the group, in this case the alkyl group, may contain 1 carbon atom, 2 carbon atoms, 3 carbon atoms, etc., up to and including 20 carbon atoms. More preferably, the alkyl is a medium size alkyl having 1 to 10 carbon atoms. Most preferably, unless otherwise indicated, the alkyl is a lower alkyl having 1 to 4 carbon atoms. The alkyl group may be substituted or unsubstituted. Substituted alkyl may have one or more substituents, whereby each substituent group can independently be, for example, hydroxyalkyl, trihaloalkyl, cycloalkyl, alkenyl, alkynyl, aryl, heteroaryl, heteroalicyclic, amine, halo, sulfonate, sulfoxide, phosphonate, hydroxy, alkoxy, aryloxy, thiohydroxy, thioalkoxy, thioaryloxy, cyano, nitro, azo, azido, sulfonamide, C-carboxylate, O-carboxylate, N-thiocarbamate, O-thiocarbamate, urea, thiourea, N-carbamate, O-carbamate, C-amide, N-amide, guanyl, guanidine and hydrazine.

The alkyl group can be an end group, as this phrase is defined hereinabove, wherein it is attached to a single adjacent atom, or a linking moiety, as this phrase is defined hereinabove, which connects two or more moieties via at least two carbons in its chain. When an alkyl is a linking moiety, it is also referred to herein as "alkylene", e.g., methylene, ethylene, propylene, etc.

The term "alkenyl" describes an unsaturated alkyl, as defined herein, having at least two carbon atoms and at least one carbon-carbon double bond. The alkenyl may be substituted or unsubstituted by one or more substituents, as described for alkyl hereinabove.

The terms "alkynyl" or "alkyne", as defined herein, is an unsaturated alkyl having at least two carbon atoms and at least one carbon-carbon triple bond. The alkynyl may be substituted or unsubstituted by one or more substituents, as described hereinabove.

The term "cycloalkyl" describes an all-carbon monocyclic or fused ring (i.e., rings that share an adjacent pair of carbon atoms) group where one or more of the rings does not have a completely conjugated pi-electron system. The cycloalkyl group may be substituted or unsubstituted. Substituted cycloalkyl may have one or more substituents, whereby each substituent group can independently be, for example, hydroxyalkyl, trihaloalkyl, cycloalkyl, alkenyl, alkynyl, aryl, heteroaryl, heteroalicyclic, amine, halo, sulfonate, sulfoxide, phosphonate, hydroxy, alkoxy, aryloxy, thiohydroxy, thioalkoxy, thioaryloxy, cyano, nitro, azo, azido, sulfonamide, C-carboxylate, O-carboxylate, N-thiocarbamate, O-thiocarbamate, urea, thiourea, N-carbamate, O-carbamate, C-amide, N-amide, guanyl, guanidine and hydrazine. The cycloalkyl group can be an end group, as this phrase is defined hereinabove, wherein it is attached to a single adjacent atom, or a linking moiety, as this phrase is defined hereinabove, connecting two or more moieties at two or more positions thereof.

The term "heteroalicyclic" describes a monocyclic or fused ring group having in the ring(s) one or more atoms such as nitrogen, oxygen and sulfur. The rings may also have one or more double bonds. However, the rings do not have a completely conjugated pi-electron system. The heteroalicyclic may be substituted or unsubstituted. Substituted heteroalicyclic may have one or more substituents, whereby each substituent group can independently be, for example, hydroxyalkyl, trihaloalkyl, cycloalkyl, alkenyl, alkynyl, aryl, heteroaryl, heteroalicyclic, amine, halo, sulfonate, sulfoxide, phosphonate, hydroxy, alkoxy, aryloxy, thiohydroxy, thioalkoxy, thioaryloxy, cyano, nitro, azo, azido, sulfonamide, C-carboxylate, O-carboxylate, N-thiocarbamate, O-thiocarbamate, urea, thiourea, O-carbamate, N-carbamate, C-amide, N-amide, guanyl, guanidine and hydrazine. The heteroalicyclic group can be an end group, as this phrase is defined hereinabove, where it is attached to a single adjacent atom, or a linking moiety, as this phrase is defined hereinabove, connecting two or more moieties at two or more positions thereof. Representative examples are piperidine, piperazine, tetrahydrofurane, tetrahydropyrane, morpholino and the like.

The term "aryl" describes an all-carbon monocyclic or fused-ring polycyclic (i.e., rings which share adjacent pairs of carbon atoms) groups having a completely conjugated pi-electron system. The aryl group may be substituted or unsubstituted. Substituted aryl may have one or more substituents, whereby each substituent group can independently be, for example, hydroxyalkyl, trihaloalkyl, cycloalkyl, alkenyl, alkynyl, aryl, heteroaryl, heteroalicyclic, amine, halo, sulfonate, sulfoxide, phosphonate, hydroxy, alkoxy, aryloxy, thiohydroxy, thioalkoxy, thioaryloxy, cyano, nitro, azo, azido, sulfonamide, C-carboxylate, O-carboxylate, N-thiocarbamate, O-thiocarbamate, urea, thiourea, N-carbamate, O-carbamate, C-amide, N-amide, guanyl, guanidine and hydrazine. The aryl group can be an end group, as this term is defined hereinabove, wherein it is attached to a single adjacent atom, or a linking moiety, as this term is defined hereinabove, connecting two or more moieties at two or more positions thereof. Preferably, the aryl is phenyl.

The term "heteroaryl" describes a monocyclic or fused ring (i.e., rings which share an adjacent pair of atoms) group having in the ring(s) one or more atoms, such as, for example, nitrogen, oxygen and sulfur and, in addition, having a completely conjugated pi-electron system. Examples, without limitation, of heteroaryl groups include pyrrole, furane, thiophene, imidazole, oxazole, thiazole, pyrazole, pyridine, pyrimidine, quinoline, isoquinoline and purine. The heteroaryl group may be substituted or unsubstituted. Substituted heteroaryl may have one or more substituents, whereby each substituent group can independently be, for example, hydroxyalkyl, trihaloalkyl, cycloalkyl, alkenyl, alkynyl, aryl, heteroaryl, heteroalicyclic, amine, halo, sulfonate, sulfoxide, phosphonate, hydroxy, alkoxy, aryloxy, thiohydroxy, thioalkoxy, thioaryloxy, cyano, nitro, azo, azido, sulfonamide, C-carboxylate, O-carboxylate, N-thiocarbamate, O-thiocarbamate, urea, thiourea, O-carbamate, N-carbamate, C-amide, N-amide, guanyl, guanidine and hydrazine. The heteroaryl group can be an end group, as this phrase is defined hereinabove, where it is attached to a single adjacent atom, or a linking moiety, as this phrase is defined hereinabove, connecting two or more moieties at two or more positions thereof. Representative examples are pyridine, pyrrole, oxazole, indole, purine and the like.

The term "alkaryl" describes an alkyl, as defined herein, which is substituted by one or more aryl or heteroaryl groups. An example of alkaryl is benzyl.

The term "amine-oxide" describes a -N(OR')(R") or a -N(OR')- group, where R' and R" are as defined herein. This term refers to a -N(OR')(R") group in cases where the amine-oxide is an end group, as this phrase is defined hereinabove, and to a -N(OR')- group in cases where the amine-oxime is an end group, as this phrase is defined hereinabove.

As used herein, the term "acyl" refers to a group having the general formula -C(=O)R', -C(=O)OR', -C(=O)-O-C(=O)R', -C(=O)SR', -C(=O)N(R')₂, -C(=S)R', -C(= S)N(R')₂, and -C(=S)S(R'), -C(=NR')R", -C(=NR')OR", -C(=NR')SR", and -C(=NR')N(R")₂, wherein R' and R" are each independently hydrogen, halo, substituted or unsubstituted hydroxyl, substituted or unsubstituted thiol, substituted or unsubstituted amine, substituted or unsubstituted acyl, cyclic or acyclic, substituted or unsubstituted, branched or unbranched aliphatic, cyclic or acyclic, substituted or unsubstituted, branched or unbranched heteroaliphatic, cyclic or acyclic, substituted or unsubstituted, branched or unbranched alkyl, cyclic or acyclic, substituted or unsubstituted, branched or unbranched alkenyl, substituted or unsubstituted alkynyl, substituted or unsubstituted aryl, substituted or unsubstituted heteroaryl, aliphaticoxy, heteroaliphaticoxy, alkyloxy, heteroalkyloxy, aryloxy, heteroaryloxy, aliphaticthioxy, heteroaliphaticthioxy, alkylthioxy, heteroalkylthioxy, arylthioxy, heteroarylthioxy, mono- or di-aliphaticamino, mono- or di-heteroaliphaticamino, mono- or di-alkylamino, mono- or di-heteroalkylamino, mono- or di-arylamino, or mono- or di-heteroarylamino; or two R^{X1} groups taken together form a 5- to 6-membered heterocyclic ring. Exemplary acyl groups include aldehydes (-CHO), carboxylic acids (-CO₂H), ketones, acyl halides, esters, amides, imines, carbonates, carbamates, and ureas. Acyl substituents include, but are not limited to, any of the substituents described herein, that result in the formation of a stable moiety (e.g., aliphatic, alkyl, alkenyl, alkynyl, heteroaliphatic, heterocyclic, aryl, heteroaryl, acyl, oxo, imino, thioxo, cyano, isocyano, amino, azido, nitro, hydroxyl, thiol, halo, aliphaticamino, heteroaliphaticamino, alkylamino, heteroalkylamino, arylamino, heteroarylamino, alkylaryl, arylalkyl, aliphaticoxy, heteroaliphaticoxy, alkyloxy, heteroalkyloxy, aryloxy, heteroaryloxy, aliphaticthioxy, heteroaliphaticthioxy, alkylthioxy, heteroalkylthioxy, arylthioxy, heteroarylthioxy, acyloxy, and the like, each of which may or may not be further substituted).

As used herein, the term "aliphatic" or "aliphatic group" denotes an optionally substituted hydrocarbon moiety that may be straight-chain (i.e., unbranched), branched, or cyclic ("carbocyclic") and may be completely saturated or may contain one or more units of unsaturation, but which is not aromatic. Unless otherwise specified, aliphatic groups contain 1-12 carbon atoms (C₁₋₁₂). In some embodiments, aliphatic groups contain 1-6 carbon atoms (C₁₋₆). In some embodiments, aliphatic groups contain 1-4 carbon atoms (C₁₋₄), and in yet other embodiments, aliphatic groups contain 1-3 carbon atoms (C₁₋₃). Suitable aliphatic groups include, but are not limited to, linear or branched, alkyl, alkenyl, and alkynyl groups, and hybrids thereof such as (cycloalkyl)alkyl, (cycloalkenyl)alkyl or (cycloalkyl)alkenyl.

As used herein, the terms "heteroaliphatic" or "heteroaliphatic group", denote an optionally substituted hydrocarbon moiety having, in addition to carbon atoms, from one to five heteroatoms, that may be straight-chain (i.e., unbranched), branched, or cyclic ("heterocyclic") and may be completely saturated or may contain one or more units of unsaturation, but which is not aromatic. Unless otherwise specified, heteroaliphatic groups contain 1-6 carbon atoms wherein 1-3 carbon atoms are optionally and independently replaced with heteroatoms selected from oxygen, nitrogen and sulfur. In some embodiments, heteroaliphatic groups contain 1-4 carbon atoms, wherein 1-2 carbon atoms are optionally and independently replaced with heteroatoms selected from oxygen, nitrogen and sulfur. In yet other embodiments, heteroaliphatic groups contain 1-3 carbon atoms, wherein 1 carbon atom is optionally and independently replaced with a heteroatom selected from oxygen, nitrogen and sulfur. Suitable heteroaliphatic groups include, but are not limited to, linear or branched, heteroalkyl, heteroalkenyl, and heteroalkynyl groups.

The term "halo" describes fluorine, chlorine, bromine or iodine substituent.

The term "halide" describes an anion of a halogen atom, namely F⁻, Cl⁻ Br⁻ and I⁻.

The term "haloalkyl" describes an alkyl group as defined above, further substituted by one or more halide.

The term "sulfate" describes a -O-S(=O)₂-OR' end group, as this term is defined hereinabove, or an -O-S(=O)₂-O- linking moiety, as these phrases are defined hereinabove, where R' is as defined hereinabove.

The term "thiosulfate" describes a -O-S(=S)(=O)-OR' end group or a -O-S(=S)(=O)-O-linking moiety, as these phrases are defined hereinabove, where R' is as defined hereinabove.

The term "sulfite" describes an -O-S(=O)-O-R' end group or a -O-S(=O)-O- group linking moiety, as these phrases are defined hereinabove, where R' is as defined hereinabove.

The term "thiosulfite" describes a -O-S(=S)-O-R' end group or an -O-S(=S)-O- group linking moiety, as these phrases are defined hereinabove, where R' is as defined hereinabove.

The term "sulfinate" or "sulfinyl" describes a -S(=O)-OR' end group or an -S(=O)-O-group linking moiety, as these phrases are defined hereinabove, where R' is as defined hereinabove.

The terms "solfoxide" or "sulfinyl" describe a -S(=O)R' end group or an -S(=O)- linking moiety, as these phrases are defined hereinabove, where R' is as defined hereinabove.

The term "sulfonate" or "sulfonyl" describes a -S(=O)₂-R' end group or an -S(=O)₂-linking moiety, as these phrases are defined hereinabove, where R' is as defined herein.

The term "S-sulfonamide" describes a -S(=O)₂-NR'R" end group or a -S(=O)₂-NR'-linking moiety, as these phrases are defined hereinabove, with R' and R'' as defined herein.

The term "N-sulfonamide" describes an R'S(=O)₂-NR"- end group or a -S(=O)₂-NR'-linking moiety, as these phrases are defined hereinabove, where R' and R'' are as defined herein.

The term "disulfide" refers to a -S-SR' end group or a -S-S- linking moiety, as these phrases are defined hereinabove, where R' is as defined herein.

The term "phosphate" describes an -O-P(=O)₂(OR') end or reactive group or a -O-P(=O)₂(O)- linking moiety, as these phrases are defined hereinabove, with R' as defined herein.

The term "phosphonate" describes a -P(=O)(OR')(OR") end or reactive group or a -P(=O)(OR')(O)- linking moiety, as these phrases are defined hereinabove, with R' and R" as defined herein.

The term "thiophosphonate" describes a -P(=S)(OR')(OR") end group or a -P(=S)(OR')(O)- linking moiety, as these phrases are defined hereinabove, with R' and R" as defined herein.

The term "carbonyl" or "carbonate" as used herein, describes a -C(=O)-R' end group or a -C(=O)- linking moiety, as these phrases are defined hereinabove, with R' as defined herein.

The term "thiocarbonyl" as used herein, describes a -C(=S)-R' end group or a -C(=S)-linking moiety, as these phrases are defined hereinabove, with R' as defined herein.

The term "oxo" as used herein, described a =O end group.

The term "thioxo" as used herein, described a =S end group.

The term "oxime" describes a =N-OH end group or a =N-O- linking moiety, as these phrases are defined hereinabove.

The term "hydroxyl" describes a -OH group.

As used herein, the term "aldehyde" refers to an -C(=O)-H group.

The term "acyl halide" describes a -(C=O)R"" group wherein R"" is halo, as defined hereinabove.

The term "alkoxy" as used herein describes an -O-alkyl, an -O-cycloalkyl, as defined hereinabove. The ether group -O- is also a possible linking moiety.

The term "aryloxy" describes both an -O-aryl and an -O-heteroaryl group, as defined herein.

The term "disulfide" as used herein describes an -S-S- linking moiety, which in some cases forms between two thiohydroxyl groups.

The terms "thio", "sulfhydryl" or "thiohydroxyl" as used herein describe an -SH group.

The term "thioalkoxy" or "thioether" describes both a -S-alkyl group, and a -S-cycloalkyl group, as defined herein. The thioether group -S- is also a possible linking moiety.

The term "thioaryloxy" describes both a -S-aryl and a -S-heteroaryl group, as defined herein. The thioarylether group -S-aryl- is also a possible linking moiety.

The term "cyano" or "nitrile" describes a -C≡N group.

The term "isocyanate" describes an -N=C=O group.

The term "nitro" describes an -NO₂ group.

The term "carboxylate" or "ester", as used herein encompasses C-carboxylate and O-carboxylate.

The term "C-carboxylate" describes a -C(=O)-OR' end group or a -C(=O)-O- linking moiety, as these phrases are defined hereinabove, where R' is as defined herein.

The term "O-carboxylate" describes a -OC(=O)R' end group or a -OC(=O)- linking moiety, as these phrases are defined hereinabove, where R' is as defined herein.

The term "thiocarboxylate" as used herein encompasses "C-thiocarboxylate and O-thiocarboxylate.

The term "C-thiocarboxylate" describes a -C(=S)-OR' end group or a -C(=S)-O- linking moiety, as these phrases are defined hereinabove, where R' is as defined herein.

The term "O-thiocarboxylate" describes a -OC(=S)R' end group or a -OC(=S)- linking moiety, as these phrases are defined hereinabove, where R' is as defined herein.

The term "carbamate" as used herein encompasses N-carbamate and O-carbamate.

The term "N-carbamate" describes an R"OC(=O)-NR'- end group or a -OC(=O)-NR'-linking moiety, as these phrases are defined hereinabove, with R' and R" as defined herein.

The term "O-carbamate" describes an -OC(=O)-NR'R" end group or an -OC(=O)-NR'- linking moiety, as these phrases are defined hereinabove, with R' and R" as defined herein.

The term "thiocarbamate" as used herein encompasses N-thiocarbamate and O-thiocarbamate.

The term "O-thiocarbamate" describes a -OC(=S)-NR'R" end group or a -OC(=S)-NR'- linking moiety, as these phrases are defined hereinabove, with R' and R" as defined herein.

The term "N-thiocarbamate" describes an R"OC(=S)NR'- end group or a -OC(=S)NR'-linking moiety, as these phrases are defined hereinabove, with R' and R" as defined herein.

The term "dithiocarbamate" as used herein encompasses N-dithiocarbamate and S-dithiocarbamate.

The term "S-dithiocarbamate" describes a -SC(=S)-NR'R" end group or a -SC(=S)NR'- linking moiety, as these phrases are defined hereinabove, with R' and R" as defined herein.

The term "N-dithiocarbamate" describes an R"SC(=S)NR'- end group or a -SC(=S)NR'-linking moiety, as these phrases are defined hereinabove, with R' and R" as defined herein.

The term "urea", which is also referred to herein as "ureido", describes a -NR'C(=O)-NR"R‴ end group or a -NR'C(=O)-NR"- linking moiety, as these phrases are defined hereinabove, where R' and R" are as defined herein and R‴ is as defined herein for R' and R".

The term "thiourea", which is also referred to herein as "thioureido", describes a -NR'-C(=S)-NR"R‴ end group or a -NR'-C(=S)-NR"- linking moiety, with R', R" and R‴ as defined herein.

The term "amide" as used herein encompasses C-amide and N-amide.

The term "C-amide" describes a -C(=O)-NR'R" end group or a -C(=O)-NR'- linking moiety, as these phrases are defined hereinabove, where R' and R" are as defined herein.

The term "N-amide" describes a R'C(=O)-NR"- end group or a R'C(=O)-N- linking moiety, as these phrases are defined hereinabove, where R' and R" are as defined herein.

The term "imine", which is also referred to in the art interchangeably as "Schiff-base", describes a -N=CR'- linking moiety, with R' as defined herein or hydrogen. As is well known in the art, Schiff bases are typically formed by reacting an aldehyde or a ketone and an amine-containing moiety such as amine, hydrazine, hydrazide and the like, as these terms are defined herein. The term "aldimine" refers to a -CH=N- imine which is derived from an aldehyde. The term "ketimine" refers to a -CR'=N- imine which is derived from a ketone.

The term "hydrazone" refers to a -R'C=N-NR"- linking moiety, wherein R' and R" are as defined herein.

The term "semicarbazone" refers to a linking moiety which forms in a condensation reaction between an aldehyde or ketone and semicarbazide. A semicarbazone linking moiety stemming from a ketone is a -R'C=NNR"C(=O)NR"'-, and a linking moiety stemming from an aldehyde is a -CR'=NNR"C(=O)NR"'-, wherein R' and R" are as defined herein and R‴ or as defined for R'.

As used herein, the term "lactone" refers to a cyclic ester, namely the intra-condensation product of an alcohol group -OH and a carboxylic acid group -COOH in the same molecule.

As used herein, the term "lactam" refers to a cyclic amide, as this term is defined herein. A lactam with two carbon atoms beside the carbonyl and four ring atoms in total is referred to as a β-lactam, a lactam with three carbon atoms beside the carbonyl and five ring atoms in total is referred to as a γ-lactam, a lactam with four carbon atoms beside the carbonyl and six ring atoms in total is referred to as a δ-lactam, and so on.

The term "guanyl" describes a R'R"NC(=N)- end group or a -R'NC(=N)- linking moiety, as these phrases are defined hereinabove, where R' and R" are as defined herein.

The term "guanidine" describes a -R'NC(=N)-NR"R‴ end group or a - R'NC(=N)- NR"- linking moiety, as these phrases are defined hereinabove, where R', R" and R‴ are as defined herein.

The term "hydrazine" describes a -NR'-NR"R‴ end group or a -NR'-NR"- linking moiety, as these phrases are defined hereinabove, with R', R", and R‴ as defined herein.

As used herein, the term "hydrazide" describes a -C(=O)-NR'-NR"R‴ end group or a - C(=O)-NR'-NR"- linking moiety, as these phrases are defined hereinabove, where R', R" and R‴ are as defined herein.

The term "hydroxylamine", as used herein, refers to either a -NHOH group or a -ONH₂.

As used herein, the terms "azo" or "diazo" describe a -N=N-R' end group or a -N=N-linking moiety, as these phrases are defined hereinabove, where R' is as defined herein.

As used herein, the term "azido" described a -N=N⁺=N⁻ (-N₃) end group.

The term "triazine" refers to a heterocyclic ring, analogous to the six-membered benzene ring but with three carbons replaced by nitrogen atoms. The three isomers of triazine are distinguished from each other by the positions of their nitrogen atoms, and are referred to as 1,2,3-triazine, 1,2,4-triazine, and 1,3,5-triazine. Other aromatic nitrogen heterocycles include pyridines with 1 ring nitrogen atom, diazines with 2 nitrogen atoms in the ring and tetrazines with 4 ring nitrogen atoms.

The term "triazole" refers to either one of a pair of isomeric chemical compounds with molecular formula C₂H₃N₃, having a five-membered ring of two carbon atoms and three nitrogen atoms, namely 1,2,3-triazoles and 1,2,4-triazoles.

The term "aziridine", as used herein, refers to a reactive group which is a three membered heterocycle with one amine group and two methylene groups, having a molecular formula of - C₂H₃NH.

As used herein, the term "thiohydrazide" describes a -C(=S)-NR'-NR"R‴ end group or a -C(=S)-NR'-NR"- linking moiety, as these phrases are defined hereinabove, where R', R" and R‴ are as defined herein.

As used herein, the term "methyleneamine" describes an -NR'-CH₂-CH=CR"R‴ end group or a -NR'-CH₂-CH=CR"- linking moiety, as these phrases are defined hereinabove, where R', R" and R'" are as defined herein.

The term "diene", as used herein, refers to a -CR'=CR"-CR"'=CR""- group, wherein R' as defined hereinabove, and R", R‴ and R"" are as defined for R'.

The term "dienophile", as used herein, refers to a reactive group that reacts with a diene, typically in a Diels-Alder reaction mechanism, hence a dienophile is typically a double bond or an alkenyl.

The term "epoxy", as used herein, refers to a reactive group which is a three membered heterocycle with one oxygen and two methylene groups, having a molecular formula of -C₂H₃O.

According to some embodiments of the present invention, some linking moieties result from a reaction between two reactive groups. Alternatively, a desired linking moiety is first generated and a bioactive agent and/or a spacer moiety are attached thereto.

### Cleavable linker lability:

According to some embodiments of the present invention, the conjugate exhibits a linking moiety that is stable at physiological conditions, namely a linking moiety that does not disintegrate spontaneously or otherwise for the duration of exposure to the physiological environment in the bodily site. Such linking moiety is referred to herein a "biostable". Biostable linking moieties offer the advantage of an extended period of time at which the conjugate can exert its biological activity, up to the time it is secreted or otherwise removed from the bodily site. An exemplary biostable linking moiety is a triazole-based linking moiety. It is noted that biostability is also a relative term, meaning that a biostable linking moiety takes longer to break or requires certain cleavage conditions which hare less frequently encountered by the conjugate when present in physiological conditions.

According to some embodiments of the present invention, the linking moiety is a cleavable linker, or a biocleavable-linking moiety. In the context of some embodiments of the present invention, the linking moiety is a cleavable linker, or biocleavable linking moiety, which is selected so as to break and release the bioactive agent attached thereto at certain conditions, referred to herein as "drug-releasing conditions" or "cleavage conditions". As used herein, the terms "biocleavable" and "biodegradable" are used interchangeably to refer to moieties that degrade (i.e., break and/or lose at least some of their covalent structure) under physiological or endosomal conditions. Biodegradable moieties are not necessarily hydrolytically degradable and may require enzymatic action to degrade.

As used herein, the terms "cleavable linker", "biocleavable moiety" or "biodegradable moiety" describe a chemical moiety, which undergoes cleavage in a biological system such as, for example, the digestive system of an organism or a metabolic system in a living cell.

According to some embodiments of the present invention, the linking moiety is a photocleavable linker that cleaves upon light irradiation.

In some embodiments, the cleavable linker is selected according to its susceptibility to certain enzymes that are likely to be present at the targeted bodily site or at any other bodily site where cleavage is intended, thereby defining the cleavage conditions.

Representative examples of biocleavable moieties include, without limitation, amides, carboxylates, carbamates, phosphates, hydrazides, thiohydrazides, disulfides, epoxides, peroxo and methyleneamines. Such moieties are typically subjected to enzymatic cleavages in a biological system, by enzymes such as, for example, hydrolases, amidases, kinases, peptidases, phospholipases, lipases, proteases, esterases, epoxide hydrolases, nitrilases, glycosidases and the like.

For example, hydrolases (EC number beginning with 3) catalyze hydrolysis of a chemical bond according to the general reaction scheme A-B + H₂O → A-OH + B-H. Ester bonds are cleaved by sub-group of hydrolases known as esterases (EC number beginning with 3.1), which include nucleases, phosphodiesterases, lipases and phosphatases. Hydrolases having an EC number beginning with 3.4 are peptidases, which act on peptide bonds.

Additional information pertaining to enzymes, enzymatic reactions, and enzyme-linking moiety correlations can be found in various publically accessible sources, such as Bairoch A., "The ENZYME database in 2000", Nucleic Acids Res, 2000, 28, pp. 304-305.

### Functional moiety:

As disclosed herein, the conjugate is designed to comprise a payload, or carry a releasable payload in the form of a dolastatin 10 analog and a functional moiety, derived from a bioactive agent, a diagnostic agent or a labeling agent. In embodiments in which the moieties are of agents are different from one-another, the conjugate of the present invention provides for simultaneous, concerted or sequential release of the agents and can therefore be specifically advantageous in cases where the different agents confer a cumulative and/or a synergistic effect, or when one agent serves as the targeting agent for the other, bringing the payload to an intended target.

The term "functional moiety", as used in the context of embodiments of the present invention, refer to any bioactive, diagnostic or labeling moiety, such as, without limitation, an amino-acid, a peptide, a polypeptide, a protein, an antibody, a biopolymer, a targeting agent, a drug, a dye, a nanoparticle, a bead, and the likes. The term "functional moiety" relates to the term "bioactive agent" while considering that a biologic activity, a diagnostic activity and/or an imaging activity, effected or exerted *in vitro* and/or *in vivo,* is generally regarded as a biologic activity. In the context of the present embodiments, the terms "bioactive agent", and "pharmaceutically active agent" are used interchangeably. In some embodiments the bioactive agent is a drug.

As used herein, in some embodiments, the terms "functional moiety", "bioactive agent" and "drug" refer to small molecules or large or small biomolecules/polymers that alter, inhibit, activate, or otherwise affect a biological mechanism or event. Bioactive agent that can be tethered to the conjugate, according to embodiments of the present invention, include, but are not limited to, anti-cancer substances for all types and stages of cancer and cancer treatments (chemotherapeutic, proliferative, acute, genetic, spontaneous etc.), anti-proliferative agents, photosensitizing agents, chemosensitizing agents, anti-inflammatory agents (including steroidal and non-steroidal anti-inflammatory agents and anti-pyretic agents), antimicrobial agents (including antibiotics, antiviral, antifungal, anti-parasite, anti-protozoan etc.), anti-oxidants, hormones, anti-hypertensive agents, anti-AIDS substances, anti-diabetic substances, immunosuppressants, enzyme inhibitors, neurotoxins, opioids, hypnotics, anti-histamines, lubricants, tranquilizers, anti-convulsants, muscle relaxants and anti-Parkinson substances, antipruritic agents, anti-spasmodics and muscle contractants including channel blockers, miotics and anti-cholinergics, anti-glaucoma compounds, modulators of cell-extracellular matrix interactions including cell growth inhibitors and anti-adhesion molecules, vitamins, vasodilating agents, inhibitors of DNA, RNA or protein synthesis, analgesics, anti-angiogenic factors, anti-secretory factors, anticoagulants and/or anti-thrombotic agents, anesthetics, ophthalmics, prostaglandins, anti-depressants, anti-psychotic substances, anti-emetics, radioactive agents and imaging agents. A more comprehensive listing of exemplary drugs suitable for use in the present invention may be found in "Pharmaceutical Substances: Syntheses, Patents, Applications" by Axel Kleemann and Jurgen Engel, Thieme Medical Publishing, 1999; the "Merck Index: An Encyclopedia of Chemicals, Drugs, and Biologicals", edited by Susan Budavari et al., CRC Press, 1996, and the United States Pharmacopeia-25/National Formulary-20, published by the United States Pharmcopeial Convention, Inc., Rockville Md., 2001.

As used herein, the term "small molecule" refers to molecules, whether naturally-occurring or artificially created (e.g., via chemical synthesis), that have a relatively low molecular weight. Typically, small molecules are monomeric and have a molecular weight of less than about 1500 Da. Preferred small molecules are biologically active in that they produce a local or systemic effect in animals, preferably mammals, more preferably humans. In certain preferred embodiments, the small molecule is a drug. Preferably, though not necessarily, the drug is one that has already been deemed safe and effective for use by the appropriate governmental agency or body. For example, drugs for human use listed by the FDA under 21 C.F.R. §§330.5, 331 through 361, and 440 through 460; drugs for veterinary use listed by the FDA under 21 C.F.R. §§500 through 589, are all considered acceptable for use in accordance with the present invention.

Anti-cancer drugs that can be linked and controllably released from the conjugate according to some embodiments of the invention include, but are not limited to Chlorambucil; 3-(9-Acridinylamino)-5-(hydroxymethyl)aniline; Azatoxin; Acivicin; Aclarubicin; Acodazole Hydrochloride; Acronine; Adriamycin; Adozelesin; Aldesleukin; Altretamine; Ambomycin; Ametantrone Acetate; Aminoglutethimide; Amsacrine; Anastrozole; Anthramycin; Asparaginase; Asperlin; Azacitidine; Azetepa; Azotomycin; Batimastat; Benzodepa; Bicalutamide; Bisantrene Hydrochloride; Bisnafide Dimesylate; Bizelesin; Bleomycin Sulfate; Brequinar Sodium; Bropirimine; Busulfan; Cactinomycin; Calusterone; Caracemide; Carbetimer; Carboplatin; Carmustine; Carubicin Hydrochloride; Carzelesin; Cedefingol; Cirolemycin; Cisplatin; Cladribine; Crisnatol Mesylate; Cyclophosphamide; Cytarabine; Dacarbazine; Dactinomycin; Daunorubicin Hydrochloride; Decitabine; Dexormaplatin; Dezaguanine; Dezaguanine Mesylate; Diaziquone; Docetaxel; Doxorubicin; Doxorubicin Hydrochloride; Droloxifene; Droloxifene Citrate; Dromostanolone Propionate; Duazomycin; Edatrexate; Eflornithine Hydrochloride; Elsamitrucin; Enloplatin; Enpromate; Epipropidine; Epirubicin Hydrochloride; Erbulozole; Esorubicin Hydrochloride; Estramustine; Estramustine Phosphate Sodium; Etanidazole; Etoposide; Etoposide Phosphate; Etoprine; Fadrozole Hydrochloride; Fazarabine; Fenretinide; Floxuridine; Fludarabine Phosphate; Fluorouracil; Flurocitabine; Fosquidone; Fostriecin Sodium; Gemcitabine; Gemcitabine Hydrochloride; Hydroxyurea; Idarubicin Hydrochloride; Ifosfamide; Ilmofosine; Interferon Alfa-2a; Interferon Alfa-2b; Interferon Alfa-n1; Interferon Alfa-n3; Interferon Beta- I a; Interferon Gamma- I b; Iproplatin; Irinotecan Hydrochloride; Lanreotide Acetate; Letrozole; Leuprolide Acetate; Liarozole Hydrochloride; Lometrexol Sodium; Lomustine; Losoxantrone Hydrochloride; Masoprocol; Maytansine; Mechlorethamine Hydrochloride; Megestrol Acetate; Melengestrol Acetate; Melphalan; Menogaril; Mercaptopurine; Methotrexate; Methotrexate Sodium; Metoprine; Meturedepa; Mitindomide; Mitocarcin; Mitocromin; Mitogillin; Mitomalcin; Mitomycin; Mitosper; Mitotane; Mitoxantrone Hydrochloride; Mycophenolic Acid; Nocodazole; Nogalamycin; Ormaplatin; Oxisuran; Paclitaxel; Pegaspargase; Peliomycin; Pentamustine; Peplomycin Sulfate; Perfosfamide; Pipobroman; Piposulfan; Piroxantrone Hydrochloride; Plicamycin; Plomestane; Porfimer Sodium; Porfiromycin; Prednimustine; Procarbazine Hydrochloride; Puromycin; Puromycin Hydrochloride; Pyrazofurin; Riboprine; Rogletimide; Safingol; Safingol Hydrochloride; Semustine; Simtrazene; Sparfosate Sodium; Sparsomycin; Spirogermanium Hydrochloride; Spiromustine; Spiroplatin; Streptonigrin; Streptozocin; Sulofenur; Talisomycin; Taxol; Tecogalan Sodium; Tegafur; Teloxantrone Hydrochloride; Temoporfin; Teniposide; Teroxirone; Testolactone; Thiamiprine; Thioguanine; Thiotepa; Tiazofuirin; Tirapazamine; Topotecan Hydrochloride; Toremifene Citrate; Trestolone Acetate; Triciribine Phosphate; Trimetrexate; Trimetrexate Glucuronate; Triptorelin; Tubulozole Hydrochloride; Uracil Mustard; Uredepa; Vapreotide; Verteporfin; Vinblastine Sulfate; Vincristine Sulfate; Vindesine; Vindesine Sulfate; Vinepidine Sulfate; Vinglycinate Sulfate; Vinleurosine Sulfate; Vinorelbine Tartrate; Vinrosidine Sulfate; Vinzolidine Sulfate; Vorozole; Zeniplatin; Zinostatin; Zorubicin Hydrochloride. Additional antineoplastic agents include those disclosed in Chapter 52, Antineoplastic Agents (Paul Calabresi and Bruce A. Chabner), and the introduction thereto, 1202-1263, of Goodman and Gilman's "The Pharmacological Basis of Therapeutics", Eighth Edition, 1990, McGraw-Hill, Inc. (Health Professions Division).

Non-limiting examples of chemotherapeutic agents that can be efficiently delivered by the conjugates of the present invention, include amino containing chemotherapeutic agents such as camptothecin, daunorubicin, doxorubicin, N-(5,5-diacetoxypentyl)doxorubicin, anthracycline, mitomycin C, mitomycin A, 9-amino aminopertin, antinomycin, N⁸-acetyl spermidine, 1-(2-chloroethyl)-1,2-dimethanesulfonyl hydrazine, bleomycin, tallysomucin, and derivatives thereof; hydroxy containing chemotherapeutic agents such as etoposide, irinotecan, topotecan, 9-amino camptothecin, paclitaxel, docetaxel, esperamycin, 1,8-dihydroxy-bicyclo[7.3.1]trideca-4-ene-2,6-diyne-13-one, anguidine, morpholino-doxorubicin, vincristine and vinblastine, and derivatives thereof, sulfhydril containing chemotherapeutic agents and carboxyl containing chemotherapeutic agents. Additional chemotherapeutic agents include, without limitation, an alkylating agent such as a nitrogen mustard, an ethylenimine and a methylmelamine, an alkyl sulfonate, a nitrosourea, and a triazene; an antimetabolite such as a folic acid analog, a pyrimidine analog, and a purine analog; a natural product such as a vinca alkaloid, an epipodophyllotoxin, an antibiotic, an enzyme, a taxane, and a biological response modifier; miscellaneous agents such as a platinum coordination complex, an anthracenedione, an anthracycline, a substituted urea, a methyl hydrazine derivative, or an adrenocortical suppressant; or a hormone or an antagonist such as an adrenocorticosteroid, a progestin, an estrogen, an antiestrogen, an androgen, an antiandrogen, a gonadotropin-releasing hormone analog, bleomycin, doxorubicin, paclitaxel, 4-OH cyclophosphamide and cisplatinum.

Anti-inflammatory drugs that can be linked and controllably released from the conjugate according to some embodiments of the invention include, but are not limited to Alclofenac; Alclometasone Dipropionate; Algestone Acetonide; Alpha Amylase; Amcinafal; Amcinafide; Amfenac Sodium; Amiprilose Hydrochloride; Anakinra; Anirolac; Anitrazafen; Apazone; Balsalazide Disodium; Bendazac; Benoxaprofen; Benzydamine Hydrochloride; Bromelains; Broperamole; Budesonide; Carprofen; Cicloprofen; Cintazone; Cliprofen; Clobetasol Propionate; Clobetasone Butyrate; Clopirac; Cloticasone Propionate; Cormethasone Acetate; Cortodoxone; Deflazacort; Desonide; Desoximetasone; Dexamethasone Dipropionate; Diclofenac Potassium; Diclofenac Sodium; Diflorasone Diacetate; Diflumidone Sodium; Diflunisal; Difluprednate; Diftalone; Dimethyl Sulfoxide; Drocinonide; Endrysone; Enlimomab; Enolicam Sodium; Epirizole; Etodolac; Etofenamate; Felbinac; Fenamole; Fenbufen; Fenclofenac; Fenclorac; Fendosal; Fenpipalone; Fentiazac; Flazalone; Fluazacort; Flufenamic Acid; Flumizole; Flunisolide Acetate; Flunixin; Flunixin Meglumine; Fluocortin Butyl; Fluorometholone Acetate; Fluquazone; Flurbiprofen; Fluretofen; Fluticasone Propionate; Furaprofen; Furobufen; Halcinonide; Halobetasol Propionate; Halopredone Acetate; Ibufenac; Ibuprofen; Ibuprofen Aluminum; Ibuprofen Piconol; Ilonidap; Indomethacin; Indomethacin Sodium; Indoprofen; Indoxole; Intrazole; Isoflupredone Acetate; Isoxepac; Isoxicam; Ketoprofen; Lofemizole Hydrochloride; Lomoxicam; Loteprednol Etabonate; Meclofenamate Sodium; Meclofenamic Acid; Meclorisone Dibutyrate; Mefenamic Acid; Mesalamine; Meseclazone; Methylprednisolone Suleptanate; Momiflumate; Nabumetone; Naproxen; Naproxen Sodium; Naproxol; Nimazone; Olsalazine Sodium; Orgotein; Orpanoxin; Oxaprozin; Oxyphenbutazone; Paranyline Hydrochloride; Pentosan Polysulfate Sodium; Phenbutazone Sodium Glycerate; Pirfenidone; Piroxicam; Piroxicam Cinnamate; Piroxicam Olamine; Pirprofen; Prednazate; Prifelone; Prodolic Acid; Proquazone; Proxazole; Proxazole Citrate; Rimexolone; Romazarit; Salcolex; Salnacedin; Salsalate; Sanguinarium Chloride; Seclazone; Sermetacin; Sudoxicam; Sulindac; Suprofen; Talmetacin; Talniflumate; Talosalate; Tebufelone; Tenidap; Tenidap Sodium; Tenoxicam; Tesicam; Tesimide; Tetrydamine; Tiopinac; Tixocortol Pivalate; Tolmetin; Tolmetin Sodium; Triclonide; Triflumidate; Zidometacin; and Zomepirac Sodium.

Suitable antimicrobial agents, including antibacterial, antifungal, antiprotozoal and antiviral agents, for use in context of the present invention include, without limitation, beta-lactam drugs, quinolone drugs, ciprofloxacin, norfloxacin, tetracycline, erythromycin, amikacin, triclosan, doxycycline, capreomycin, chlorhexidine, chlortetracycline, oxytetracycline, clindamycin, ethambutol, metronidazole, pentamidine, gentamicin, kanamycin, lineomycin, methacycline, methenamine, minocycline, neomycin, netilmicin, streptomycin, tobramycin, and miconazole. Also included are tetracycline hydrochloride, farnesol, erythromycin estolate, erythromycin stearate (salt), amikacin sulfate, doxycycline hydrochloride, chlorhexidine gluconate, chlorhexidine hydrochloride, chlortetracycline hydrochloride, oxytetracycline hydrochloride, clindamycin hydrochloride, ethambutol hydrochloride, metronidazole hydrochloride, pentamidine hydrochloride, gentamicin sulfate, kanamycin sulfate, lineomycin hydrochloride, methacycline hydrochloride, methenamine hippurate, methenamine mandelate, minocycline hydrochloride, neomycin sulfate, netilmicin sulfate, paromomycin sulfate, streptomycin sulfate, tobramycin sulfate, miconazole hydrochloride, amanfadine hydrochloride, amanfadine sulfate, triclosan, octopirox, parachlorometa xylenol, nystatin, tolnaftate and clotrimazole and mixtures thereof.

Non-limiting examples of anti-oxidants that are usable in the context of the present invention include ascorbic acid (vitamin C) and its salts, ascorbyl esters of fatty acids, ascorbic acid derivatives (e.g., magnesium ascorbyl phosphate, sodium ascorbyl phosphate, ascorbyl sorbate), tocopherol (vitamin E), tocopherol sorbate, tocopherol acetate, other esters of tocopherol, butylated hydroxy benzoic acids and their salts, 6-hydroxy-2,5,7,8-tetramethylchroman-2-carboxylic acid (commercially available under the trade name Trolox^{R}), gallic acid and its alkyl esters, especially propyl gallate, uric acid and its salts and alkyl esters, sorbic acid and its salts, lipoic acid, amines (e.g., N,N-diethylhydroxylamine, amino-guanidine), sulfhydryl compounds (e.g., glutathione), dihydroxy fumaric acid and its salts, lycine pidolate, arginine pilolate, nordihydroguaiaretic acid, bioflavonoids, curcumin, lysine, methionine, proline, superoxide dismutase, silymarin, tea extracts, grape skin/seed extracts, melanin, and rosemary extracts.

Non-limiting examples of vitamins usable in context of the present invention include vitamin A and its analogs and derivatives: retinol, retinal, retinyl palmitate, retinoic acid, tretinoin, iso-tretinoin (known collectively as retinoids), vitamin E (tocopherol and its derivatives), vitamin C (L-ascorbic acid and its esters and other derivatives), vitamin B₃ (niacinamide and its derivatives), alpha hydroxy acids (such as glycolic acid, lactic acid, tartaric acid, malic acid, citric acid, etc.) and beta hydroxy acids (such as salicylic acid and the like).

Non-limiting examples of antihistamines usable in context of the present invention include chlorpheniramine, brompheniramine, dexchlorpheniramine, tripolidine, clemastine, diphenhydramine, promethazine, piperazines, piperidines, astemizole, loratadine and terfenadine.

Representative examples of hormones include, without limitation, methyltestosterone, androsterone, androsterone acetate, androsterone propionate, androsterone benzoate, androsteronediol, androsteronediol-3-acetate, androsteronediol-17-acetate, androsteronediol 3-17-diacetate, androsteronediol-17-benzoate, androsteronedione, androstenedione, androstenediol, dehydroepiandrosterone, sodium dehydroepiandrosterone sulfate, dromostanolone, dromostanolone propionate, ethylestrenol, fluoxymesterone, nandrolone phenpropionate, nandrolone decanoate, nandrolone furylpropionate, nandrolone cyclohexane-propionate, nandrolone benzoate, nandrolone cyclohexanecarboxylate, androsteronediol-3-acetate-1-7-benzoate, oxandrolone, oxymetholone, stanozolol, testosterone, testosterone decanoate, 4-dihydrotestosterone, 5α-dihydrotestosterone, testolactone, 17α-methyl-19-nortestosterone and pharmaceutically acceptable esters and salts thereof, and combinations of any of the foregoing.

Non-limiting examples of analgesic agents that can be efficiently delivered by the conjugates of the present invention, include acetaminophen, alfentanil hydrochloride, aminobenzoate potassium, aminobenzoate sodium, anidoxime, anileridine, anileridine hydrochloride, anilopam hydrochloride, anirolac, antipyrine, aspirin, benoxaprofen, benzydamine hydrochloride, bicifadine hydrochloride, brifentanil hydrochloride, bromadoline maleate, bromfenac sodium, buprenorphine hydrochloride, butacetin, butixirate, butorphanol, butorphanol tartrate, carbamazepine, carbaspirin calcium, carbiphene hydrochloride, carfentanil citrate, ciprefadol succinate, ciramadol, ciramadol hydrochloride, clonixeril, clonixin, codeine, codeine phosphate, codeine sulfate, conorphone hydrochloride, cyclazocine, dexoxadrol hydrochloride, dexpemedolac, dezocine, diflunisal, dihydrocodeine bitartrate, dimefadane, dipyrone, doxpicomine hydrochloride, drinidene, enadoline hydrochloride, epirizole, ergotamine tartrate, ethoxazene hydrochloride, etofenamate, eugenol, fenoprofen, fenoprofen calcium, fentanyl citrate, floctafenine, flufenisal, flunixin, flunixin meglumine, flupirtine maleate, fluproquazone, fluradoline hydrochloride, flurbiprofen, hydromorphone hydrochloride, ibufenac, indoprofen, ketazocine, ketorfanol, ketorolac tromethamine, letimide hydrochloride, levomethadyl acetate, levomethadyl acetate hydrochloride, levonantradol hydrochloride, levorphanol tartrate, lofemizole hydrochloride, lofentanil oxalate, lorcinadol, lornoxicam, magnesium salicylate, mefenamic acid, menabitan hydrochloride, meperidine hydrochloride, meptazinol hydrochloride, methadone hydrochloride, methadyl acetate, methopholine, methotrimeprazine, metkephamid acetate, mimbane hydrochloride, mirfentanil hydrochloride, molinazone, morphine sulfate, moxazocine, nabitan hydrochloride, nalbuphine hydrochloride, nalmexone hydrochloride, namoxyrate, nantradol hydrochloride, naproxen, naproxen sodium, naproxol, nefopam hydrochloride, nexeridine hydrochloride, noracymethadol hydrochloride, ocfentanil hydrochloride, octazamide, olvanil, oxetorone fumarate, oxycodone, oxycodone hydrochloride, oxycodone terephthalate, oxymorphone hydrochloride, pemedolac, pentamorphone, pentazocine, pentazocine hydrochloride, pentazocine lactate, phenazopyridine hydrochloride, phenyramidol hydrochloride, picenadol hydrochloride, pinadoline, pirfenidone, piroxicam olamine, pravadoline maleate, prodilidine hydrochloride, profadol hydrochloride, propiram fumarate, propoxyphene hydrochloride, propoxyphene napsylate, proxazole, proxazole citrate, proxorphan tartrate, pyrroliphene hydrochloride, remifentanil hydrochloride, salcolex, salethamide maleate, salicylamide, salicylate meglumine, salsalate, sodium salicylate, spiradoline mesylate, sufentanil, sufentanil citrate, talmetacin, talniflumate, talosalate, tazadolene succinate, tebufelone, tetrydamine, tifurac sodium, tilidine hydrochloride, tiopinac, tonazocine mesylate, tramadol hydrochloride, trefentanil hydrochloride, trolamine, veradoline hydrochloride, verilopam hydrochloride, volazocine, xorphanol mesylate, xylazine hydrochloride, zenazocine mesylate, zomepirac sodium and zucapsaicin.

Non-limiting examples of photosensitizers include photofrin, photoporphyrin, benzoporphyrin, tookad, antrin, purlytin, foscan, and halogenated dyes disclosed, e.g. in U.S. Patent Nos 9,572,881, 9,040,721, 8,962,797, 8,748,446 and EP2850061.

According to some preferred embodiments of the present invention, the conjugate includes a somatostatin analog as a functional moiety. In other preferred embodiments, the conjugate includes a dolastatin 10 analog linked via a -CH₂- on its thiazole moiety, and a octreotide moiety, conjugated via a disulfide linking moiety. Exemplary conjugates, according to some embodiments of the present invention, include without limitation conjugate **8b,** conjugate **8c,** or conjugate **8d,** which have been prepared and their biological activity characterized and analysed, as demonstrated in the Examples section that follows below.

### Targeting moiety:

In some embodiments, the functional moiety is a moiety of a bioactive agent which exhibits an affinity to a specific biologic/chemical target, and therefore it is referred to herein as a targeting moiety. As used herein, the term "targeting moiety" describes a molecular entity that exhibits an affinity to a desired molecular or bodily site (e.g., particular organ, cells, tissues, organelles, biopolymers, receptors or the likes). In some embodiments, a targeting moiety is specific to certain targets. The target is typically a biomolecule that occurs at a higher concentration or exclusively at the targeted bodily site. In some embodiments, the targeting moiety is a biomolecule or a derivative thereof that has a specific and relatively high affinity to the target.

Targeting moieties are often employed as the bimolecular carrier in order to direct a drug to specific structures in the body or sites of physiological functions. According to some embodiments, a targeting moiety is a compound with structure or site specific reactivity.

Exemplary targeting agents include, without limitation, peptides, proteins, porphyrins, hormones, antigens, haptens, antibodies and fragments thereof, DNA fragments, RNA fragments and analogs and derivatives thereof, and any receptor ligands that bind to receptors that are expressed specifically or more abundantly at the targeted bodily sites.

As used herein, the term "biomolecule" refers to molecules (e.g., polypeptides, amino acids, polynucleotides, nucleotides, polysaccharides, sugars, lipids, nucleoproteins, glycoproteins, lipoproteins, steroids, metabolites, etc.) whether naturally-occurring or artificially created (e.g., by synthetic or recombinant methods) that are commonly found in cells and tissues. Specific classes of biomolecules include, but are not limited to, enzymes, receptors, neurotransmitters, hormones, cytokines, cell response modifiers such as growth factors and chemotactic factors, antibodies, vaccines, haptens, toxins, interferons, ribozymes, anti-sense agents, plasmids, DNA, and RNA.

In some embodiments, a targeting moiety comprises a cell-internalizing moiety, such that the molecular structure can more readily penetrate a targeted cell. Exemplary cell-internalizing moieties include, without limitation, positively charges (at physiological environment) moieties such as guanidines and amines, and moieties containing same (e.g., arginine and lysine).

In some embodiments, the targeting moiety exhibits a specific affinity to cancerous cells and neoplastic tissues. Such targeting moieties may be used to target the molecular structure presented herein, thereby delivering anticancerous bioactive agents, according to some embodiments of the present invention, to cancerous cells and tissues. The result is an enhanced effect and an improved exposure of the cancerous cells and neoplastic tissues to the anticancerous bioactive agent, preferably accompanied by reduced exposure of non-cancerous cells to the anticancerous bioactive agents.

A class of compounds that is suitable as targeting moieties, according to some embodiments of the present invention, are short peptides and peptide analogs, generally referred to herein as peptidomimetic compounds, that display more favorable pharmacological properties than their prototype native peptides. The native peptide itself, the pharmacological properties of which have been optimized, generally serves as a lead for the development of these peptidomimetics. In general, a small number of amino acids (usually four to eight) are responsible for the biological activity (recognition and binding; targeting) of a peptide ligand (targeting moiety) by a receptor (target). Once this biologically active site is determined, a lead structure for development of peptidomimetic can be optimized, for example by molecular modeling programs. U.S. Patent Nos. 5,811,392, 6,407,059 and 7,084,244 describe the preparation and use of a class of cyclic peptidomimetic targeting moieties, which can be used in the context of some embodiments of the present invention.

Peptide nucleic acid (PNA) constitute an exemplary class of targeting moiety that may be used in the context of some embodiments of the present invention. U.S. Patent No. 6,395,474 describes PNA as an analogue of DNA in which the phosphodiester backbone of DNA is replaced with a pseudo-peptide such as N-(2-amino-ethyl)-glycine. Methylenecarbonyl linkers attach DNA, RNA, or synthetic nucleobases to the polyamide backbone. PNA, obeying Watson-Crick hydrogen bonding rules, mimics the behavior of DNA and RNA by binding to complementary nucleic acid sequences such as those found in DNA, RNA, and other PNAs. An exemplary molecular structure utilizing PNA, according to some embodiments of the present invention, may bind, for example, to a specific mutated nucleic acid sequence found in the DNA of a cancerous tumor.

One example of a class of targeting moieties, which can be used advantageously in the context of embodiments of the present invention, is the family of tumor-targeting moieties that bind selectively to αᵥβ₃ and αᵥβ₅ integrins, referred to herein as the RGD (Arg-Gly-Asp) family [Arap, W. et al., Science, 1998, 279(5349):377-80]. Short peptides and peptidomimetic analogs, which are based on the RGD motif and exhibit is biological binding activity, can be used as targeting moieties in a molecular structure, according to some embodiments of the present invention, to inhibit the growth and possibly eradicate tumors in the treatment of cancer.

Additional targeting moieties, which can be used effectively in the context of the molecular structures presented herein for treating cancer, are described in the literature [e.g., *"*Novel Oncology Therapeutics: Targeted Drug Delivery for Cancer", Journal of Drug Delivery, Vol. 2013, 2013].

Non-limiting examples of targeting moieties which are useful in the context of some embodiments of the present invention include octreotide (OCT), lanreotide, pasireotide, vapreotide, cilengitide analog c(RGDfK), and luteinizing Hormone-Releasing Hormone (LHRH), bombesin, and arginine-glycine-aspartic acid (RGD).

### Advantageous process of preparation:

According to embodiments of the present invention, the dolastatin 10 analog can be prepared using standard and/or modified solid-state peptide synthesis (SSPS) techniques and reagents, as demonstrated in the Examples section that follows. In addition to the dolastatin 10 analog, some of the functional moieties contemplated above and demonstrated below, can also be prepared by SSPS techniques and reagents, as also demonstrated in the Examples section that follows.

The use of SSPS allows diversification of the product, namely allows relatively simple exploration of analogs space aiming at discovering new compounds with improved bioactivity and ADME-Tox properties, and optimization thereof. For example, since the conjugate, according to some embodiments of the present invention, may comprise a number of naturally occurring and/or synthetic amino-acid residues, as well as other conjugate elements that are built into the structure by SSPS, such as the linker, it would be relatively simple to form a family of analogous conjugates using standard or high-throughput SSPS techniques.

Hence, according to some embodiments of the present invention, there is provided a process for producing the dolastatin 10 analog provided herein, using SSPS approach. The process may include a step of attaching a first peptide unit of the analog to a solid support resin, and thereafter adding the next peptide units sequentially before releasing the resulting molecule from the resin. The first peptide unit of the dolastatin 10 analog to be attached the resin may be an amine-protected variant of 2-(1-(^{λ2}-azaneyl)-2-phenylethyl)-5/6-(A-B-substituted)-thiazole, such as **BU-5,** presented hereinbelow. The following peptide units to be coupled to the first unit may be an amine-protected derivative of (2R,3R)-3-methoxy-2-methyl-3-((S)-pyrrolidin-2-yl)propanoic acid **(BU-1),** followed by an amine-protected derivative of (3R,4S,5S)-3-methoxy-5-methyl-4-(methylamino)heptanoic acid **(BU-2),** followed by an amine-protected derivative of Val-OH, followed by dimethyl-Val to afford Dolas-OL after cleaving the nascent peptide from the resin.

In embodiments wherein the conjugate comprises a peptide-like functional moiety, the same approach h may be applied, starting from constructing the functional moiety or from the dolastatin 10 analog side, and working the way down the sequence to afford a complete conjugate.

The linker moiety may also be part of the SSPS process, or alternatively, the two major moiety of the conjugate may be synthesized separately, by SSPS or otherwise, and thereafter be coupled to form a linking moiety.

### Medical conditions:

The dolastatin 10 analog presented herein can be used to treat any medical condition that is treatable by dolastatin 10, by administration of a therapeutically effective amount of the dolastatin 10 analog to a subject in need thereof. The analog, according to some embodiments of the present invention, can also be used to prepare a pharmaceutical composition comprising the same and a pharmaceutically acceptable carrier and other optional ingredients. Thus, the analog provided herein can be used as an active ingredient in a method of treating any medical condition that is treatable by dolastatin 10, by administering a therapeutically effective amount thereof to the subject in need thereof.

The conjugate presented herein can be used to treat any medical condition that is treatable by administration of a bioactive agent (drug) with dolastatin 10 or the analog thereof, according to some embodiments of the present invention. According to some embodiments of the present invention, it is advantageous to use the conjugate to treat medical conditions, which are treatable by administration of a combination of drugs. In some embodiments, the medical condition includes an autoimmune disease, a genetic disease, a degenerative disease, a psychiatric or mental disease or condition. In some embodiments, the medical condition includes a peptic ulcer disease, Alzheimer's disease, rheumatoid arthritis, post-traumatic stress disorder, Crohn's disease, tuberculosis, leprosy, malaria and HIV/AIDS.

According to some embodiments, the degenerative disease includes Alzheimer's disease, Amyotrophic Lateral Sclerosis (ALS), a.k.a., Lou Gehrig's Disease, Osteoarthritis, Atherosclerosis, Cancer, Charcot Marie Tooth Disease (CMT), Chronic Obstructive Pulmonary Disease (COPD), Chronic traumatic encephalopathy, Diabetes, Ehlers-Danlos Syndrome, Essential tremor, Friedreich's ataxia, Leg Disease, Huntington's Disease, Inflammatory Bowel Disease (IBD), Keratoconus, Keratoglobus, Macular degeneration, Marfan's Syndrome, Multiple sclerosis, Multiple system atrophy, Muscular dystrophy, Niemann Pick disease, Osteoporosis, Parkinson's Disease, Progressive supranuclear palsy, Prostatitis, Retinitis Pigmentosa, Rheumatoid Arthritis, and Tay-Sachs Disease.

According to some embodiments, the autoimmune disease includes Acute Disseminated Encephalomyelitis (ADEM), Acute necrotizing hemorrhagic leukoencephalitis, Addison's disease, Agammaglobulinemia, Alopecia areata, Amyloidosis, Ankylosing spondylitis, Anti-GBM/Anti-TBM nephritis, Antiphospholipid syndrome (APS), Autoimmune angioedema, Autoimmune aplastic anemia, Autoimmune dysautonomia, Autoimmune hepatitis, Autoimmune hyperlipidemia, Autoimmune immunodeficiency, Autoimmune inner ear disease (AIED), Autoimmune myocarditis, Autoimmune oophoritis, Autoimmune pancreatitis, Autoimmune retinopathy, Autoimmune thrombocytopenic purpura (ATP), Autoimmune thyroid disease, Autoimmune urticaria, Axonal & neuronal neuropathies, Balo disease, Behcet's disease, Bullous pemphigoid, Cardiomyopathy, Castleman disease, Celiac disease, Chagas disease, Chronic fatigue syndrome, Chronic inflammatory demyelinating polyneuropathy (CIDP), Chronic recurrent multifocal ostomyelitis (CRMO), Churg-Strauss syndrome, Cicatricial pemphigoid/benign mucosal pemphigoid, Crohn's disease, Cogans syndrome, Cold agglutinin disease, Congenital heart block, Coxsackie myocarditis, CREST disease, Essential mixed cryoglobulinemia, Demyelinating neuropathies, Dermatitis herpetiformis, Dermatomyositis, Devic's disease (neuromyelitis optica), Discoid lupus, Dressler's syndrome, Endometriosis, Eosinophilic esophagitis, Eosinophilic fasciitis, Erythema nodosum, Experimental allergic encephalomyelitis, Evans syndrome, Fibromyalgia, Fibrosing alveolitis, Giant cell arteritis (temporal arteritis), Giant cell myocarditis, Glomerulonephritis, Goodpasture's syndrome, Granulomatosis with Polyangiitis (GPA) (formerly called Wegener's Granulomatosis), Graves' disease, Guillain-Barre syndrome, Hashimoto's encephalitis, Hashimoto's thyroiditis, Hemolytic anemia, Henoch-Schonlein purpura, Herpes gestationis, Hypogammaglobulinemia, Idiopathic thrombocytopenic purpura (ITP), IgA nephropathy, IgG4-related sclerosing disease, Immunoregulatory lipoproteins, Inclusion body myositis, Interstitial cystitis, Juvenile arthritis, Juvenile diabetes (Type 1 diabetes), Juvenile myositis, Kawasaki syndrome, Lambert-Eaton syndrome, Leukocytoclastic vasculitis, Lichen planus, Lichen sclerosus, Ligneous conjunctivitis, Linear IgA disease (LAD), Lupus (SLE), Lyme disease, chronic, Meniere's disease, Microscopic polyangiitis, Mixed connective tissue disease (MCTD), Mooren's ulcer, Mucha-Habermann disease, Multiple sclerosis, Myasthenia gravis, Myositis, Narcolepsy, Neuromyelitis optica (Devic's), Neutropenia, Ocular cicatricial pemphigoid, Optic neuritis, Palindromic rheumatism, PANDAS (Pediatric Autoimmune Neuropsychiatric Disorders Associated with Streptococcus), Paraneoplastic cerebellar degeneration, Paroxysmal nocturnal hemoglobinuria (PNH), Parry Romberg syndrome, Parsonnage-Turner syndrome, Pars planitis (peripheral uveitis), Pemphigus, Peripheral neuropathy, Perivenous encephalomyelitis, Pernicious anemia, POEMS syndrome, Polyarteritis nodosa, Type I, II, & III autoimmune polyglandular syndromes, Polymyalgia rheumatica, Polymyositis, Postmyocardial infarction syndrome, Postpericardiotomy syndrome, Progesterone dermatitis, Primary biliary cirrhosis, Primary sclerosing cholangitis, Psoriasis, Psoriatic arthritis, Idiopathic pulmonary fibrosis, Pyoderma gangrenosum, Pure red cell aplasia, Raynauds phenomenon, Reactive Arthritis, Reflex sympathetic dystrophy, Reiter's syndrome, Relapsing polychondritis, Restless legs syndrome, Retroperitoneal fibrosis, Rheumatic fever, Rheumatoid arthritis, Sarcoidosis, Schmidt syndrome, Scleritis, Scleroderma, Sjogren's syndrome, Sperm & testicular autoimmunity, Stiff person syndrome, Subacute bacterial endocarditis (SBE), Susac's syndrome, Sympathetic ophthalmia, Takayasu's arteritis, Temporal arteritis/Giant cell arteritis, Thrombocytopenic purpura (TTP), Tolosa-Hunt syndrome, Transverse myelitis, Type 1 diabetes, Ulcerative colitis, Undifferentiated connective tissue disease (UCTD), Uveitis, Vasculitis, Vesiculobullous dermatosis, Vitiligo and Wegener's granulomatosis (now termed Granulomatosis with Polyangiitis (GPA).

In some embodiments of the present invention, the medical condition is associated with an infection caused by a pathogenic microorganism, including a viral infection, a bacterial infection, a yeast infection, a fungal infection, a protozoan infection, a parasite-related infection and the like.

Medical conditions associated with a pathogenic microorganism include, without limitation, actinomycosis, anthrax, aspergillosis, bacteremia, bacterial, bacterial skin diseases, bartonella infections, botulism, brucellosis, burkholderia infections, campylobacter infections, candidiasis, cat-scratch disease, chlamydia infections, cholera, clostridium infections, coccidioidomycosis, cryptococcosis, dermatomycoses, dermatomycoses, diphtheria, ehrlichiosis, epidemic louse borne typhus, Escherichia coli infections, fusobacterium infections, gangrene, general infections, general mycoses, gram-negative bacterial infections, Gram-positive bacterial infections, histoplasmosis, impetigo, klebsiella infections, legionellosis, leprosy, leptospirosis, listeria infections, lyme disease, maduromycosis, melioidosis, mycobacterium infections, mycoplasma infections, necrotizing fasciitis, nocardia infections, onychomycosis, ornithosis, pneumococcal infections, pneumonia, pseudomonas infections, Q fever, rat-bite fever, relapsing fever, rheumatic fever, rickettsia infections, Rocky-mountain spotted fever, salmonella infections, scarlet fever, scrub typhus, sepsis, sexually transmitted bacterial diseases, staphylococcal infections, streptococcal infections, surgical site infection, tetanus, tick-borne diseases, tuberculosis, tularemia, typhoid fever, urinary tract infection, vibrio infections, yaws, yersinia infections, Yersinia pestis plague, zoonoses and zygomycosis.

Non-limiting examples of pathogenic fungi include genus *Absidia: Absidia corymbifera;* genus *Ajellomyces: Ajellomyces capsulatus, Ajellomyces dermatitidis;* genus *Arthroderma: Arthroderma benhamiae, Arthroderma fulvum, Arthroderma gypseum, Arthroderma incurvatum, Arthroderma otae, Arthroderma vanbreuseghemii;* genus *Aspergillus: Aspergillus flavus, Aspergillus fumigatus, Aspergillus niger;* genus *Blastomyces: Blastomyces dermatitidis;* genus *Candida: Candida albicans, Candida glabrata, Candida guilliermondii, Candida krusei, Candida parapsilosis, Candida tropicalis, Candida pelliculosa;* genus *Cladophialophora: Cladophialophora carrionii;* genus *Coccidioides: Coccidioides immitis;* genus *Cryptococcus: Cryptococcus neoformans;* genus *Cunninghamella: Cunninghamella sp.;* genus *Epidermophyton: Epidermophyton floccosum;* genus *Exophiala: Exophiala dermatitidis;* genus *Filobasidiella: Filobasidiella neoformans;* genus *Fonsecaea: Fonsecaea pedrosoi;* genus *Fusarium: Fusarium solani;* genus *Geotrichum: Geotrichum candidum;* genus *Histoplasma: Histoplasma capsulatum;* genus *Hortaea: Hortaea werneckii;* genus *Issatschenkia: Issatschenkia orientalis;* genus *Madurella: Madurella grisae;* genus *Malassezia: Malassezia furfur, Malassezia globosa, Malassezia obtusa, Malassezia pachydermatis, Malassezia restricta, Malassezia slooffiae, Malassezia sympodialis;* genus *Microsporum: Microsporum canis, Microsporum fulvum, Microsporum gypseum;* genus *Mucor: Mucor circinelloides;* genus *Nectria: Nectria haematococca;* genus *Paecilomyces: Paecilomyces variotii;* genus *Paracoccidioides: Paracoccidioides brasiliensis;* genus *Penicillium: Penicillium marneffei;* genus *Pichia, Pichia anomala, Pichia guilliermondii;* genus *Pneumocystis: Pneumocystis carinii;* genus *Pseudallescheria: Pseudallescheria boydii;* genus *Rhizopus: Rhizopus oryzae;* genus *Rhodotorula: Rhodotorula rubra;* genus *Scedosporium: Scedosporium apiospermum;* genus *Schizophyllum: Schizophyllum commune;* genus *Sporothrix: Sporothrix schenckii;* genus *Trichophyton: Trichophyton mentagrophytes, Trichophyton rubrum, Trichophyton verrucosum, Trichophyton violaceum;* and genus *Trichosporon: Trichosporon asahii, Trichosporon cutaneum, Trichosporon inkin, Trichosporon mucoides.*

Non-limiting examples of other pathogenic microorganism include *Acanthamoeba* and other free-living amoebae, *Aeromonas hydrophila, Anisakis* and related worms, *Ascaris lumbricoides, Bacillus cereus, Campylobacter jejuni, Clostridium botulinum, Clostridium perfringens, Cryptosporidium parvum, Cyclospora cayetanensis, Diphyllobothrium, Entamoeba histolytica, Eustrongylides, Giardia lamblia, Listeria monocytogenes, Nanophyetus, Plesiomonas shigelloides, Salmonella, Shigella, Staphylococcus aureus, Streptococcus, Trichuris trichiura, Vibrio cholerae, Vibrio parahaemolyticus, Vibrio vulnificus* and other vibrios, *Yersinia enterocolitica* and *Yersinia pseudotuberculosis.*

### Cancer treatment and chemotherapy:

In some embodiments of the present invention, the medical condition is associated with malignant cells and tumors, collectively referred to herein as cancer.

To date, chemotherapy remains the most common and most frequently used in cancer treatment, alone or in combination with other therapies. Currently available anticancer chemotherapies act by affecting specific molecular targets in proliferating cancer cells, leading to inhibition of essential intracellular processes such as DNA transcription, synthesis and replication.

Unfortunately anticancerous drugs are highly toxic, as they are designed to kill mammalian cells, and are therefore harmful also to normal proliferating cells resulting in debilitating and even lethal side effects. Some of these adverse effects are gastrointestinal toxicity, nausea, vomiting, and diarrhea when the epithelial lining of the intestine is affected. Other side effects include alopecia, when the hair follicles are attacked, bone marrow suppression and neutropenia due to toxicity of hematopoietic precursors. Therefore the effectiveness of currently used anticancerous drugs is dose-limited due to their toxicity to normal rapidly growing cells. The use of a conjugate according to embodiments of the present invention, can optimize the balance between the desired anticancer activity of certain anticancer drugs and their adverse side effects, by quantitative determination of the actual amount of drug released in the targeted cells.

One of the contemporary approaches in the fight against cancer is engineering of molecular targeted drugs that permeate cancer cells and specifically modulate activity of molecules that belong to signal-transduction pathways. These targets include products of frequently mutated oncogenes, such as k-Ras and other proteins that belong to tyrosine kinase signal transduction pathways. For example, Imatinib (Gleevec^{®}), is the first such drug, approved for treatment of chronic myelogenous leukemia (CML). Imatinib blocks the activity of non-receptor tyrosine kinase BCR-Abl oncogene, present in 95 % of patients with CML. Imatinib was found to be effective in the treatment of CML and certain tumors of the digestive tract. Nevertheless, as others, this new compound is not completely specific to its target; therefore side effects emerge, including severe congestive cardiac failure, pulmonary tuberculosis, liver toxicity, sweet syndrome (acute febrile neutrophilic dermatosis), leukocytosis, dermal edemas, nausea, rash and musculoskeletal pain.

Angiogenesis inhibitors are currently investigated for their use in cancer treatment and to date, one anti-angiogenetic drug, Bevacizumab (Avastin^{®}), was approved for the treatment of solid tumors in combination with standard chemotherapy. However, as in all chemotherapeutic drugs, Bevacizumab causes a number of adverse side effects such as hypertension, blood clots, neutropenia, neuropathy, proteinuria and bowel perforation.

In some embodiments, the functional moiety of the conjugates presented herein, is a targeting moiety that is responsible for the higher concentration of the conjugate at the targeted bodily site compared to non-targeted bodily sites, thereby reducing the adverse side effects associated with the toxicity of the anti-cancer drugs attached thereto. In addition, the linking moieties attached the anti-cancer drugs to the conjugate are selected such that they cleave in conditions that are present at the targeted site more so than in non-targeted sites, thereby releasing the payload of drugs at the targeted site at a higher rate compared to non-targeted sites.

In the context of some embodiments of the present invention, the term "cancer" refers, but not limited to acute lymphoblastic, acute lymphoblastic leukemia, acute lymphocytic leukemia, acute myelogenous leukemia, acute myeloid leukemia, adrenocortical carcinoma, AIDS-related lymphoma, anal cancer, appendix cancer, basal-cell carcinoma, bladder cancer, brain cancer, brainstem glioma, breast cancer, bronchial adenomas/carcinoids, Burkitt's lymphoma, carcinoid tumor, cerebellar or cerebral astrocytoma, cervical cancer, cholangiocarcinoma, chondrosarcoma, chronic lymphocytic or chronic lymphocytic leukemia, chronic myelogenous or chronic myeloid leukemia, chronic myeloproliferative disorders, colon cancer, cutaneous T-cell lymphoma, desmoplastic small round cell tumor, endometrial uterine cancer, ependymoma, esophageal cancer, Ewing's sarcoma, extracranial germ cell tumor, extragonadal germ cell tumor, extrahepatic bile duct cancer, gallbladder cancer, gastric (stomach) cancer, gastrointestinal carcinoid tumor, gastrointestinal stromal tumor (GIST), gestational trophoblastic tumor, glioma of the brain stem, hairy cell leukemia, head and neck cancer, heart cancer, hepatocellular (liver) cancer, Hodgkin lymphoma, hypopharyngeal cancer, hypothalamic and visual pathway glioma, intraocular melanoma, Islet cell carcinoma, Kaposi sarcoma, laryngeal cancer, leukaemia, lip and oral cavity cancer, liposarcoma, lymphoma, male breast cancer, malignant mesothelioma, medulloblastoma, melanoma, Merkel cell skin carcinoma, mesothelioma, metastatic squamous neck cancer, mouth cancer, multiple endocrine neoplasia syndrome, multiple myeloma, multiple myeloma/plasma cell neoplasm, mycosis fungoides, myelodysplastic/myeloproliferative diseases, nasal cavity and paranasal sinus cancer, nasopharyngeal carcinoma, neuroblastoma, non-Hodgkin lymphoma, non-melanoma skin cancer, non-small cell lung cancer, oligodendroglioma, oral cancer, oropharyngeal cancer, osteosarcoma and malignant fibrous histiocytoma, ovarian cancer, ovarian germ cell tumor, ovarian epithelial cancer (surface epithelial-stromal tumor), ovarian low malignant potential tumor, pancreatic cancer, paranasal sinus and nasal cavity cancer, parathyroid cancer, penile cancer, pharyngeal cancer, pheochromocytoma, pineal astrocytoma, pineal germinoma, pineoblastoma and supratentorial primitive neuroectodermal tumors, pituitary adenoma, plasma cell neoplasia, pleuropulmonary blastoma, primary carcinoma, primary central nervous system lymphoma, primary liver cancer, prostate cancer, rectal cancer, renal cell carcinoma, renal pelvis and ureter carcinoma, retinoblastoma, rhabdomyosarcoma, salivary gland cancer, Sézary syndrome, small cell lung cancer, small intestine cancer, soft tissue sarcoma, squamous cell carcinoma, stomach cancer, supratentorial primitive neuroectodermal tumor, testicular cancer, throat cancer, thymoma and thymic carcinoma, thyroid cancer, transitional cell cancer of the renal pelvis and ureter, urethral cancer, uterine sarcoma, vaginal cancer, visual pathway and hypothalamic glioma, vulvar cancer, Waldenström macroglobulinemia and Wilms tumor.

**As used** herein the term "about" refers to ± 10 %.

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the phrases "substantially devoid of" and/or "essentially devoid of" in the context of a certain substance, refer to a composition that is totally devoid of this substance or includes less than about 5, 1, 0.5 or 0.1 percent of the substance by total weight or volume of the composition. Alternatively, the phrases "substantially devoid of" and/or "essentially devoid of" in the context of a process, a method, a property or a characteristic, refer to a process, a composition, a structure or an article that is totally devoid of a certain process/method step, or a certain property or a certain characteristic, or a process/method wherein the certain process/method step is effected at less than about 5, 1, 0.5 or 0.1 percent compared to a given standard process/method, or property or a characteristic characterized by less than about 5, 1, 0.5 or 0.1 percent of the property or characteristic, compared to a given standard.

The term "exemplary" is used herein to mean "serving as an example, instance or illustration". Any embodiment described as "exemplary" is not necessarily to be construed as preferred or advantageous over other embodiments and/or to exclude the incorporation of features from other embodiments.

The words "optionally" or "alternatively" are used herein to mean "is provided in some embodiments and not provided in other embodiments". Any particular embodiment of the invention may include a plurality of "optional" features unless such features conflict.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the terms "process" and "method" refer to manners, means, techniques and procedures for accomplishing a given task including, but not limited to, those manners, means, techniques and procedures either known to, or readily developed from known manners, means, techniques and procedures by practitioners of the chemical, material, mechanical, computational and digital arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental and/or calculated support in the following examples.

### EXAMPLES

Reference is now made to the following examples, which together with the above descriptions illustrate some embodiments of the invention in a non-limiting fashion.

### EXAMPLE 1

### Synthesis of Dolastatin analog Dolas-Ol

For the synthesis of the exemplary dolastatin 10 analog, Dolas-Ol, on solid support (Cl-Trt resin), the thiazole **BU-5** was prepared starting from Phe amide (see, Scheme 1). **BU-4.1** was first Alloc protected then reacted with Lawesson's reagent to yield **BU-4.3.** The latter was transformed into **BU-4** using ethyl bromopyruvate followed by reduction with NaBH₄ to obtain the desired **BU-5** (allyl (S)-(1-(4-(hydroxymethyl)thiazol-2-yl)-2-phenylethyl)carbamate).

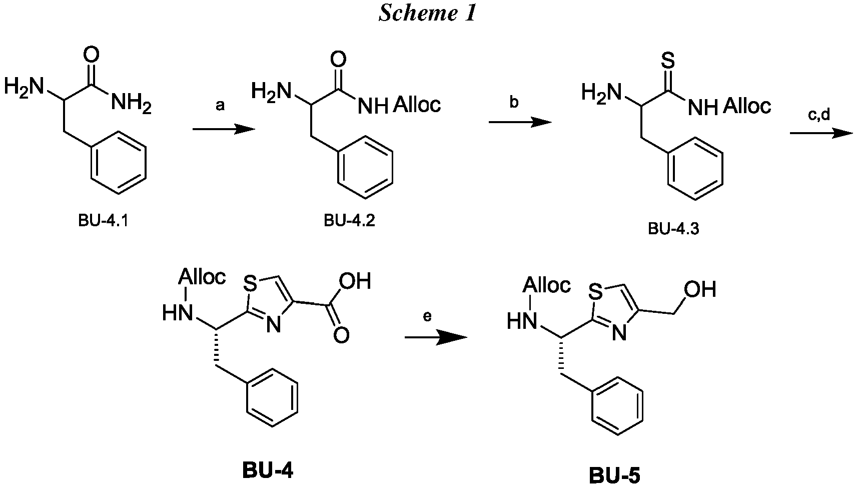

In Scheme 1, showing the synthesis of **BU-5**, 2a (**BU-1**) and 2b (**BU-2**): a. Allyl chloroformate, TEA, DCM, 0 °c.o.n; b. Lawesson's reagent, THF, reflux, 4 h; c. Ethyl bromopyruvate, EtOH, 18 h; d. KOH, EtOH. 24 h; and e. NaBH₄, TEA, Ethyl chloroformate, THF, 30 min.

The Fmoc protected version was also prepared by first Alloc deprotection (Pd Tetrakis, DMBA, DCE) and then Fmoc protection Fmoc-BU5-CO₂C₆H₄NO₂, DIEA, (Fmoc-OSu, Na₂CO₃, MeOH/H₂O), as shown in Scheme 2 below.

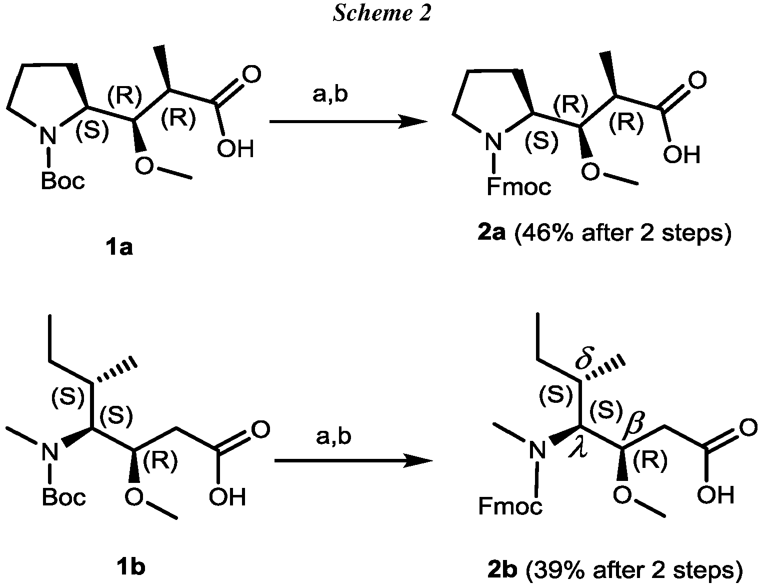

In Scheme 2: a. TFA/DCM (1:1), 30 min, 0 °C; b. Fmoc-Cl, TEA, DCM, 0 °C to rt, 10h.

Thereafter, the peptide was assembled and cleaved to afford the exemplary dolastatin 10 analog "Dolas-Ol" after standard purification in preparative HPLC. Scheme 3 below presents that solid-phase peptide synthesis (SPPS) of Dolas-Ol.

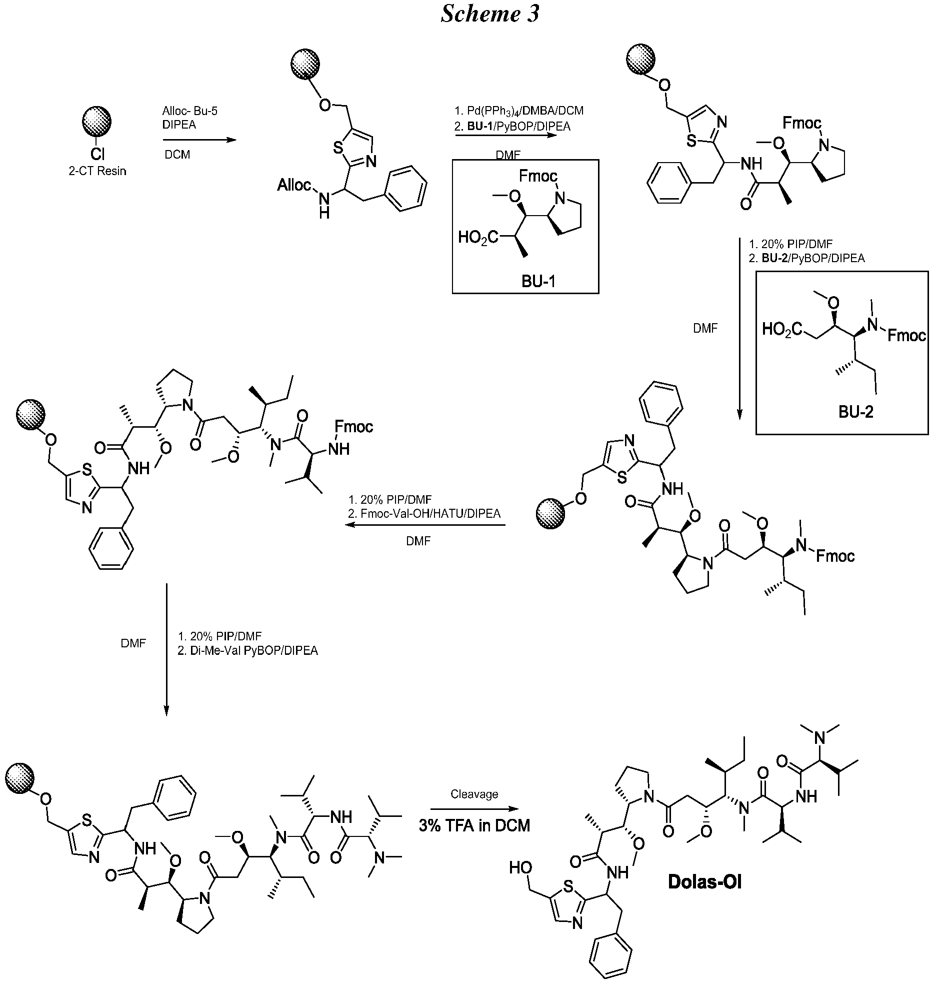

The IUPAC name of the presently disclosed dolastatin analog, referred to herein as "Dolas-Ol", is (2S)-2-((S)-2-(dimethylamino)-3-methylbutanamido)-N-((3R,4S,5S)-1-((2S)-2-((1R,2R)-3-((1-(5-(hydroxymethyl)thiazol-2-yl)-2-phenylethyl)amino)-1-methoxy-2-methyl-3-oxopropyl)pyrrolidin-1-yl)-3-methoxy-5-methyl-1-oxoheptan-4-yl)-N,3-dimethylbutanamide.

### EXAMPLE 2

### Activity of Dolastatin 10 analog

The exemplary dolastatin 10 analog, Dolas-Ol, was subjected to an *in vitro* assay to test its cytotoxicity towards two cancerous cell lines: WM266-4 (malignant melanoma cell line)) and HCT116 (human colon cancer cell line). The study was design to assess the activity of a dolastatin 10 analog, according to some embodiments of the present invention, compared to other dolastatin 10 derivatives known in the art, Auristatin F, Auristatin F-Ol and Dolastatin-CO₂H.

Briefly, MW266-4 and HC116 cells were exposed to increasing doses of MMAF and Dolas-Ol and at the end of 8 days incubation, metabolic activities of cells were measured using the XTT assay kit. All samples contained DMSO at final concentration < 0.05%. Cells cultures were initiated in microplate wells at a concentration of 2-4x10⁴ cells/well and the cells were allowed to adhere for 24 h incubation period. At this time the cells were washed, fresh medium containing different concentrations of the tested substances were added and the cells were incubated for additional 8 days. The medium was then removed, all the wells were washed with PBS and cultured for 24 hrs in fresh medium without drugs. The cells were washed again and given a fresh medium containing the XTT reagent and re-incubated for 2-4 h. Absorbence in the wells was measured with a TECAN Infinite M200 ELISA reader at both 480 and 680 nm e, the latter being the background absorbance. The absorbencies were normalized to the absorbance of cells grown in medium and solvent. All the tests were repeated three times in quadruplicates.

The results are presented in Table 1 in IC₅₀ values in nM.

**Table 1**

| Compound: | Structure: | Purity | MW266 (8 days) | HC116 (8 days) |
|---|---|---|---|---|
| Auristatin F (MMAF) | 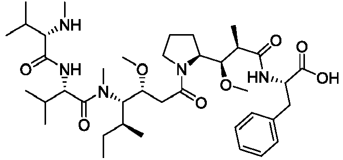 | 99% | 15.59±0.03 | 18.27±0.06 |
| Auristatin F-Ol (MMAF-OL) | 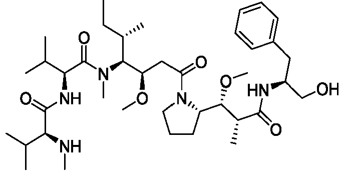 | 95% | 58.82±0.05 | 29.71±0.04 |
| Dolastatin-CO₂H | 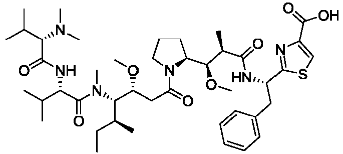 | 94% | 31.75±0.02 | 52.50±0.03 |
| Dolas-Ol **(provided herein)** | 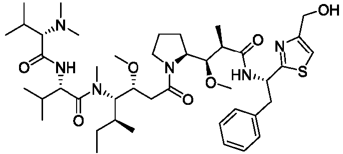 | 98% | 2.24±0.03 | 3.67±0.03 |

As can be seen in Table 1, the analog designed according to the concept presented herein (see, Formula I), appears to be the most active namely exhibit the highest cytotoxicity against cancerous cells.

### EXAMPLE 3

### Conjugates of Dolastatin analog

During the last decades somatostatin receptor family (SSTRs) constituting of 5 subtypes (SSTR 1-5) has drawn attention of medicinal chemists as promising targets for peptide-based targeted drug delivery. SSTRs are heterogeneously expressed in the variety of human solid tumors. So far, the most useful peptide developed as a drug carrier for SSTR overexpressed malignancies has been the S-S-bridged octapeptide octreotide (sandostatin) that preferentially targets SSTR2. Several drug conjugates based on octreotide have been reported bearing small molecule drugs camptothecin (CPT) and doxorubicin. However, no peptide conjugates bearing anticancer dolastatin payload were reported hitherto.

One of the objectives of the present invention is to provide a reliable and practical platform for the production of conjugates of dolastatin and peptides, such as SSTR ligands and analogs thereof. The presently disclosed platform allows, *inter alia,* to employ the fast and cost-effective solid phase synthesis of peptides and peptidomimetic building-blocks. For example, the platform allows the preparation of a C-terminus modified octreotide, namely octreotide amide, conjugated to the presently disclosed Dolas-Ol analog through various linkers. This fast and reproducible synthesis, effected mostly by solid phase chemistry, enables the production and characterization of eltire series of such conjugates in high throughput screening for rapid structure-function optimization.

Octreotide, sold under the brand name Sandostatin among others, is an octapeptide that mimics natural somatostatin pharmacologically, though it is a more potent inhibitor of growth hormone, glucagon, and insulin than the natural hormone. It was first synthesized in 1979, by the chemist Wilfried Bauer. It was approved for use in the United States in 1988. As a demonstrative example, Dolas-Ol was conjugated to the SSTR2 ligand octreotide-amide, via a biocleavable linker moiety, connecting between the N-terminus of the octreotide and the added hydroxyl group on the dolastatin analog side (see scheme below).

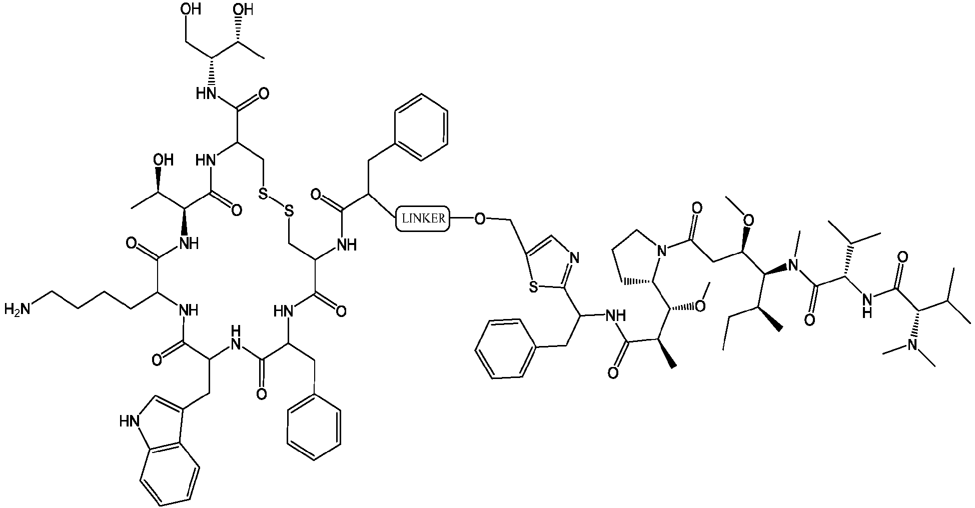

A fast and convenient Fmoc chemistry SPPS synthesis of these novel conjugates is provides herein. As exemplary embodiments, two Fmoc protected biodegradable S-S linkers 4a-b have been prepared, and the disulfide likers are presented in Scheme 4 below.

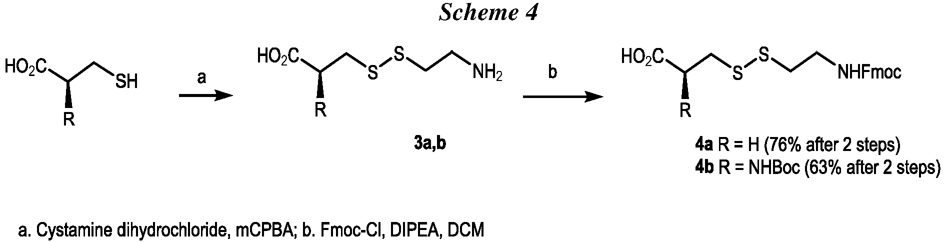

In both cases cystamine was initially reacted with oxidizing mCPBA, followed by addition of 3-mercaptopropionic acid or N-Boc-(L)-Cys to give after overnight at room temperature corresponding intermediates **3a,b** (Scheme 4). **3a,b** were further submitted to Fmoc protection with Fmoc-Cl to afford desired Fmoc protected linkers **4a,b** correspondingly ready for peptide backbone assembly by Fmoc chemistry protocol. Remarkably, the α-NH₂ group in the bifunctional linker **4b** can facilitate solubilization or used (if protected by Alloc) as an additional conjugation site of a dye moiety for theranostic applications.

Next, several amido-octreotide-Dolas-Ol conjugates were synthesized using solid phase peptide synthesis (see, Scheme 5 below). In general, the fully protected cyclic amido-octreotide peptide **5** was synthesized on an acid-labile Rink amide MBHA resin (substitution level, 0.35 mmol/g, 1g) using standard Fmoc solid-phase peptide synthesis (SPPS) by following published procedures.

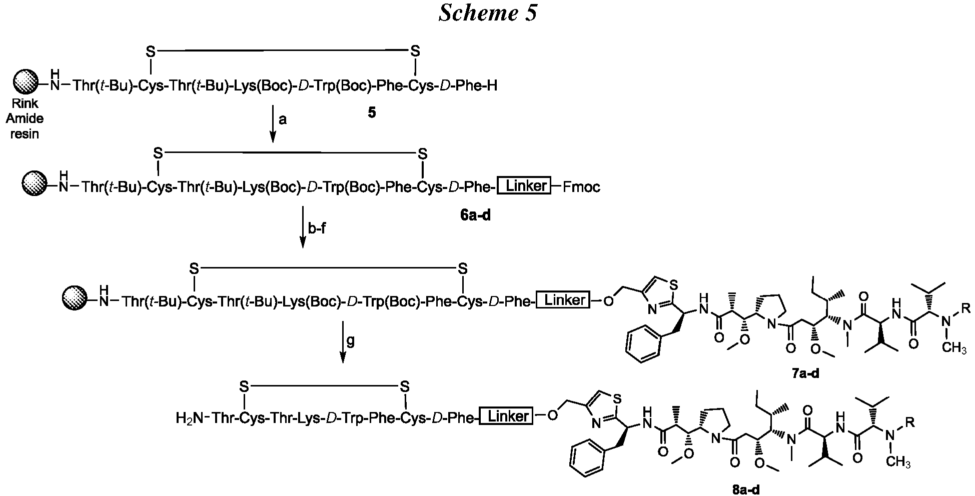

In Scheme 5, the exemplary conjugate **8a,** according to an embodiment of the present invention, exhibits the linker GABA (R=H; 41 % yield); conjugate **8b** exhibits the linker - CO(CH₂)₂S-S(CH₂)₂NH- (R=H; 38 % yield); conjugate **8c** exhibits the linker -CO(CH₂)₂S-S(CH₂)₂NH- (R=Me; 32 % yield); and the conjugate **8d** exhibits the linker -COCH(NH₂)CH₂S-S(CH₂)₂NH- (R=Me; 33 % yield).

In Scheme 5: a. (i) Fmoc-Linker-OH, PyBoP, DIEA, DMF, rt; (ii). 20 % Piperidine in DMF; b. (i) Fmoc-BU5-CO₂C₆H₄NO₂, DIEA, DMF, rt; (ii) 20 % Piperidine in DMF; c. 2a, PyBoP, DIEA, DMF, rt; (ii) 20 % Piperidine in DMF; d.(i) 2b, PyBop, DIEA, DMF, rt; (ii) 20 % Piperidine in DMF; e. (i) Fmoc-Val-OH, HATU, DIEA, DMF, rt; (ii) Fmoc-Val-OH, PyAop, DIEA, DMF, rt; (iii) 20 % Piperidine in DMF; f. Boc-(Me)Val-OH (for conjugates **8a,b**) or (Me)2Val-OH (for conjugates **8c,d**), PyBoP, DIEA, DMF, rt.; and g. 95:2.5:2.5 TFA/TIS/H2O, 0 °C then rt, 1h.

After splitting the resin-attached 5 into four reactors, each peptidyl portion was coupled to the three various Fmoc protected linkers: Fmoc-GABA, Fmoc-NH(CH₂)₂S-S(CH₂)₂CO₂H (ss linker, **4a**) and Fmoc-NH(CH₂)₂S-SCH₂CH₂(NHBoc)CO₂H (cys-ss-linker, **4b**) to yield **6a-d.** N-terminal Fmoc group of the linkers of **6a-d** was removed (20 % piperidine in NMP, 10 mL) releasing the primary amine toward consequent construction of Dolas-Ol conjugates. First Fmoc-BU5 was pre-activated with p-nitrophenyl chloroformate to afford thiazole adduct at the N-terminus. Then two amino acids (N-Fmoc- Dap and N-Fmoc-Dil) were sequentially coupled using PyBoP coupling reagent (PyBoP, DIEA, DMF, 1.5h, rt) followed by usual Fmoc removal for each amino acid. The forth amino acid Fmoc-Val was coupled twice to the free peptidyl residues using first HATU and then PyAop due to more difficult coupling to the secondary amine of Dil. The synthesis was accomplished by coupling of final amino acids Fmoc-(Me)Val to **6a,b,d** and *N,N'-*dimethyl-L-valine to **6c,** following by cleavage from the solid support (cold TFA/TIS/H₂O, 95:2.5:2.5) and affording after purification (semi-preparative HPLC) and characterization by MS the final conjugates **8a-b** respectively in good yields.

### EXAMPLE 4

### Cell cytotoxicity of Dolas-Ol conjugates

HC116, H1299 and TRAMP C2 cells that overexpressed SSTR2 were incubated for 3 hours with 1 µM doses of the conjugates, according to some embodiments of the present invention, and then the cells were washed and proceed for incubation for the following 8 days. At the end of incubation for 8 days, the metabolic activities of cells were measured using the XTT assay. The results are presented in Table 2 below.

Table 2 presents the growth inhibition values of octreotide amide, conjugates 8b-d in the cell cultures calculated according to the concentration of 1 µM. The calculation for all values is provided relative to control 100% of GI (for all values, p<0.01).

**Table 2**

| **Target cells** | **Octreotide amide** | **Conjugate 8b** | **Conjugate 8c** | **Conjugate 8d** |
|---|---|---|---|---|
| HC116 - 8 days | 6.1% | 71.8% | 69.6% | 63.1% |
| H1299 - 8 days | 8.4% | 82.7% | 66.0% | 77.8% |
| TRAMP C2 - 8 days | 6.5% | 47.8% | 72.1% | 68.4% |
| HEK as negative control. | 7.9% | 17.4% | 11.6% | 12.3% |

As can be seen in Table 2, the peptide octreotide amine alone was not cytotoxic to either cell type, while peptide drug conjugates with S-S linker **8b-d** demonstrated significant toxicities only on cell lines with overexpressed SSTR2 versus HEK with low expression.

The results demonstrate the utility of the presently disclosed exemplary dolastatin 10 analog, Dolas-Ol, exhibiting a CH_{2O}H theater at the C-terminus. Conjugates of this exemplary embodiment of a dolastatin 10 analog possess a remarkable cytotoxicity against cancer cell lines at sub-nanomolar concentration. Its selective cytotoxicity was demonstrated when conjugated to octreotide amide on SSTR 2 overexpressed cell lines vs low expressed. Thus, *Dolas-Ol* can pave a way to the novel antibody drug conjugates (ADCs) bearing highly cytotoxic payload to targeted and receptor mediated treatment of a variety of cancers.

## Claims

1. A dolastatin 10 analog have general Formula I: 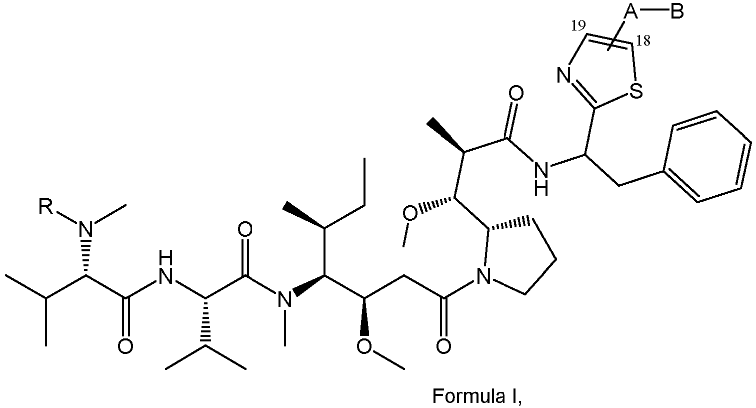 wherein:
A is a linear or branched C₁₋₆ alkyl attached to a thiazole group at position 18 or 19;
B is a functional group; and
R is an alkyl or H.

2. The dolastatin 10 analog of claim 1, wherein said linear or branched C₁₋₆ alkyl is attached at position 18 on said thiazole group.

3. The dolastatin 10 analog of any one of claims 1-2, wherein said functional group is a hydroxyl.

4. The dolastatin 10 analog of any one of claims 1-3, wherein R is methyl.

5. The dolastatin 10 analog of any one of claims 1-4, wherein A is -(CH₂)- attached at position 18, B is -OH, and the dolastatin 10 analog is having a structure: 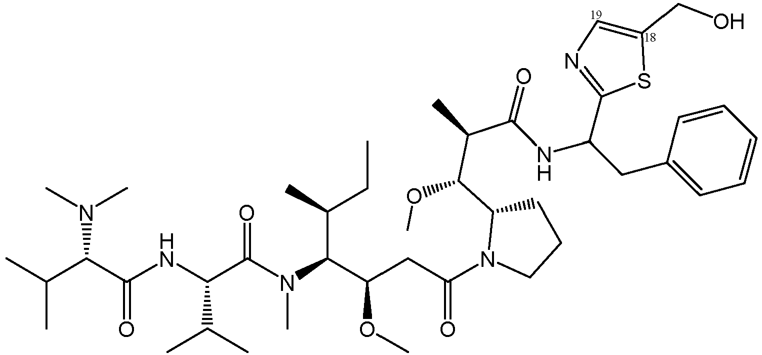 (*2S*)-2-((*S*)-2-(dimethylamino)-3-methylbutanamido)-*N*-((3*R*,4*S*,5*S*)-1-((2*S*)-2-((1*R*,2*R*)-3-((1-(5-(hydroxymethyl)thiazol-2-yl)-2-phenylethyl)amino)-1-methoxy-2-methyl-3-oxopropyl)pyrrolidin-1-yl)-3-methoxy-5-methyl-1-oxoheptan-4-yl)-*N*,3-dimethylbutanamide

6. A conjugate, comprising:
a moiety of a dolastatin 10 analog according to any one of claims 1-5,
a functional moiety selected from the group consisting of a bioactive agent, a labeling agent and/or a diagnostic agent, , and
a linking moiety connecting said dolastatin 10 analog moiety and said functional moiety, said linking moiety is optionally a biocleavable bond or a biocleavable group.

7. The conjugate of claim 6, wherein said linking moiety comprises a disulfide.

8. The conjugate claim 6, wherein said functional moiety is a somatostatin analog.

9. The conjugate claim 8, wherein said dolastatin 10 analog comprises a -CH₂-OH at its thiazole, said functional moiety is a octreotide moiety, and said linking moiety comprises a disulfide.

10. The conjugate of any one of claims 6-9, being conjugate **8b,** conjugate **8c,** or conjugate **8d:**

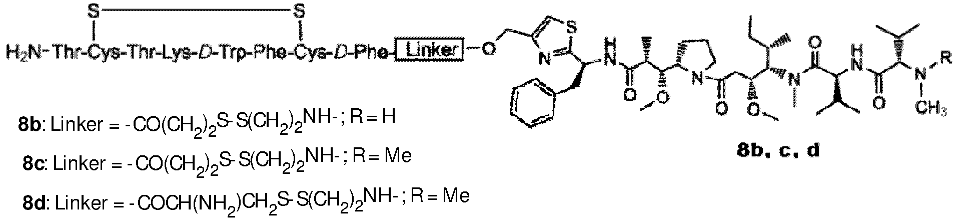

11. A process of preparing the dolastatin 10 analog of claim 5, comprising:attaching an amine-protected derivative of (2-(1-(λ²- azaneyl)-2-phenylethyl)thiazol-5-yl)methanol **(Bu-5)** to a 2-chlorotrityl chloride solid-support resin to thereby form a nascent peptide,
coupling an amine-protected derivative of (2R,3R)-3-methoxy-2-methyl-3-(*(S)*-pyrrolidin-2-yl)propanoic acid **(Bu-1)** to said nascent peptide on said resin,
coupling an amine-protected derivative of (*3R,4S,5S*)-3-methoxy-5-methyl-4-(methylamino)heptanoic acid **(Bu-2)** to said nascent peptide on said resin,
coupling an amine-protected derivative of Val-OH to said nascent peptide on said resin,
coupling dimethyl-Val to said nascent peptide on said resin, and
cleaving the dolastatin 10 analog from said resin to thereby obtain *(2S)*-2-(*(S)*-2-(dimethylamino)-3-methylbutanamido)-N-(*(3R,4S,5S)*-1-(*(2S)-2-((1R,2R)*-3-(*(1-(5-(hydroxymethyl)thiazol-2-yl)-2-*phenylethyl)amino)-1-methoxy-2-methyl-3-oxopropyl)pyrrolidin-1-yl)-3-methoxy-5-methyl-1-oxoheptan-4-yl)-N,3-dimethylbutanamide.

12. A process of preparing the conjugate of any one of claims 6-10, comprising,
forming said functional moiety on a solid-support resin using solid-state peptide synthesis protocols, wherein said functional moiety comprises a linkable group,
forming said dolastatin 10 analog starting from said linkable group, thereby forming said linking moiety on a solid-support resin using solid-state peptide synthesis protocols, and
releasing the conjugate from said resin.

13. A pharmaceutical composition comprising the dolastatin 10 analog of any one of claims 1-5, and a pharmaceutically acceptable carrier.

14. A pharmaceutical composition comprising the conjugate of any one of claims 6-10, and a pharmaceutically acceptable carrier.

## Patentansprüche

1. Dolastatin-10-Analogon, das die folgende allgemeine Formel I aufweist: 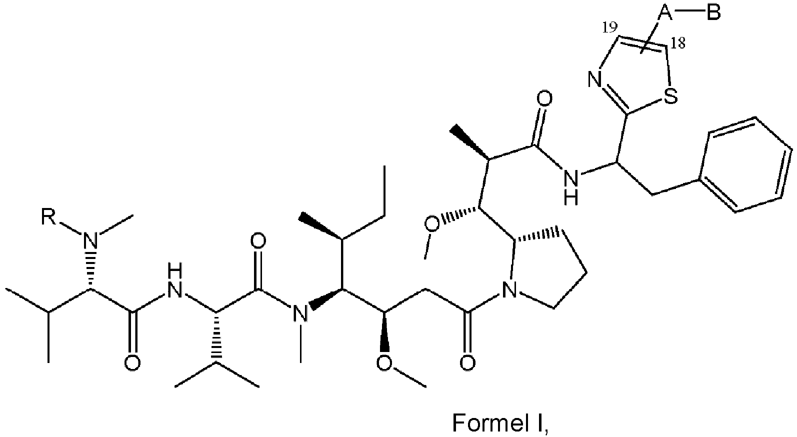 wobei:
A ein lineares oder verzweigtes C₁₋₆-Alkyl ist, das an der Position 18 oder 19 an eine Thiazolgruppe gebunden ist;
B eine funktionelle Gruppe ist; und
R ein Alkyl oder H ist.

2. Dolastatin-10-Analogon nach Anspruch 1, wobei das besagte lineare oder verzweigte C₁₋₆-Alkyl an der Position 18 an die besagte Thiazolgruppe gebunden ist.

3. Dolastatin-10-Analogon nach einem der Ansprüche 1-2, wobei die besagte funktionelle Gruppe ein Hydroxyl ist.

4. Dolastatin-10-Analogon nach einem der Ansprüche 1-3, wobei R Methyl ist.

5. Dolastatin-10-Analogon nach einem der Ansprüche 1-4, wobei A -(CH₂)- ist an der Position 18 gebunden, B -OH ist und das Dolastatin-10-Analogon eine folgende Struktur aufweist: 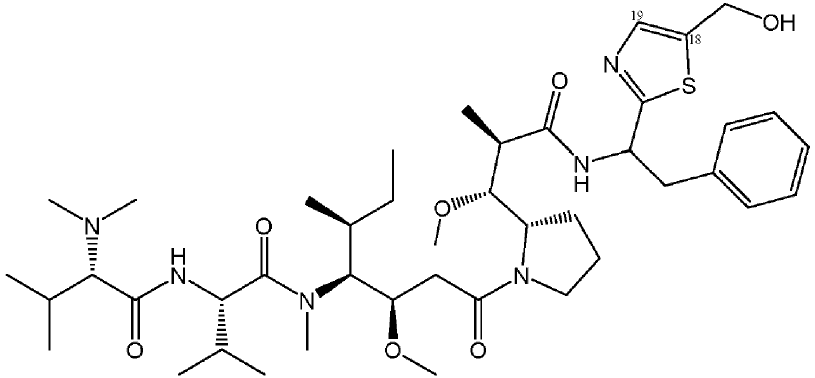 (2S)-2-((*S*)-2-(Dimethylamino)-3-methylbutanamido)-*N*-((3R,4S, 5S)-1-((2*S*)-2-((1*R*,2*R*)-3-((1-(5-(hydroxymethyl)thiazol-2-yl)-2-phenylethyl1)amino)-1-met hoxy-2-methyl-3-oxopropyl)pyrrolidin-1-yl)-3-methoxy-5-methyl-1-oxoheptan-4-yl)-N,3-dimethylbutanamid

6. Konjugat, das Folgendes umfasst:
eine Einheit eines Dolastatin-10-Analogons nach einem der Ansprüche 1-5,
eine funktionelle Einheit, die aus der Gruppe ausgewählt ist, die aus einem bioaktiven Wirkstoff, einem Markierungsmittel und/oder einem Diagnostikum besteht, und
eine Verbindungseinheit, die die besagte Dolastatin-10-Analogoneinheit und die besagte funktionelle Einheit verbindet, wobei die besagte Verbindungseinheit optional eine biologisch spaltbare Bindung oder eine biologisch spaltbare Gruppe ist.

7. Konjugat nach Anspruch 6, wobei die besagte Verbindungseinheit ein Disulfid umfasst.

8. Konjugat nach Anspruch 6, wobei die besagte funktionelle Einheit ein Somatostatin-Analogon ist.

9. Konjugat nach Anspruch 8, wobei das besagte Dolastatin-10-Analogon an seinem Thiazol -CH₂-OH umfasst, wobei die besagte funktionelle Einheit eine Octreotideinheit ist und die besagte Verbindungseinheit ein Disulfid umfasst.

10. Konjugat nach einem der Ansprüche 6-9, wobei es sich um das Konjugat 8b, das Konjugat 8c oder das Konjugat 8d handelt: 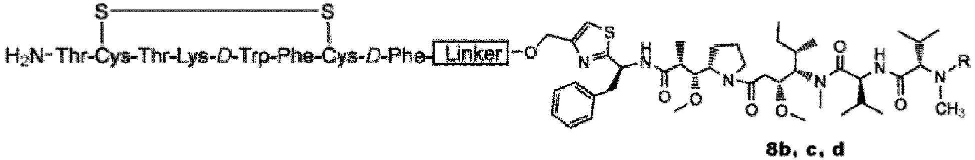
8b: Linker = -CO(CH₂)₂S-S(CH₂)₂NH-; R = H
8c: Linker = -CO(CH₂)₂S-S(CH₂)₂NH-; R = Me
8d: Linker = -COCH(NH₂)CH₂S-S(CH₂)₂NH-; R = Me

11. Verfahren zum Herstellen des Dolastatin-10-Analogons nach Anspruch 5, das Folgendes umfasst:
Anbringen eines amingeschützten Derivats von (2-(1-(λ²-azaneyl)-2-phenylethyl)thiazol-5-yl)methanol **(Bu-5)** an ein 2-Chlorotritylchlorid-Festträgerharz, um dadurch ein entstehendes Peptid zu bilden,
Koppeln eines amingeschützten Derivats der (2*R*,3*R*)-3-Methoxy-2-methyl-3-((S)-pyrrolidin-2-yl)propansäure **(Bu-1)** an das besagte entstehende Peptid auf dem besagten Harz,
Koppeln eines amingeschützten Derivats der (3*R*,4*S*,5*S*)-3-Methoxy-5-methyl-4-(methylamino)heptansäure **(Bu-2)** an das besagte entstehende Peptid auf dem besagten Harz,
Koppeln eines amingeschützten Derivats von Val-OH an das besagte entstehende Peptid auf dem besagten Harz,
Koppeln von Dimethyl-Val an das besagte entstehende Peptid auf dem besagten Harz, und
Spalten des Dolastatin-10-Analogons von dem besagten Harz, um dadurch *(2S)*-2-((S)-2-(dimethylamino)-3-methylbutanamido)-N-(*(3R,4S,5S)*-1-(*(2S)*-2-(*(1R, 2R)*-3-((1-(5-(hydroxymethyl)thiazol-2-yl)-2-phenylethyl)amino)-1-methoxy-2-methyl-3-oxopropyl)pyrrolidin-1-yl)-3-methoxy-5-methyl-1-oxoheptan-4-yl)-N,3-dimethylbuta namid zu erhalten.

12. Verfahren zum Herstellen des Konjugats nach einem der Ansprüche 6-10, das Folgendes umfasst:
Bilden der besagten funktionellen Einheit unter Verwendung von Protokollen zur Feststoff-Peptidsynthese auf einem Festträgerharz,
wobei die besagte funktionelle Einheit eine verknüpfbare Gruppe umfasst,
Bilden des besagten Dolastatin-10-Analogons ausgehend von der besagten verknüpfbaren Gruppe, wodurch die besagte Verbindungseinheit auf einem Festträgerharz unter Verwendung von Protokollen zur Feststoff-Peptidsynthese gebildet wird, und
Freigeben des Konjugats von dem besagten Harz.

13. Pharmazeutische Zusammensetzung, die das Dolastatin-10-Analogon nach einem der Ansprüche 1-5 und einen pharmazeutisch verträglichen Träger umfasst.

14. Pharmazeutische Zusammensetzung, die das Konjugat nach einem der Ansprüche 6-10 und einen pharmazeutisch verträglichen Träger umfasst.

## Revendications

1. Analogue de la dolastatine 10 ayant la formule générale I : 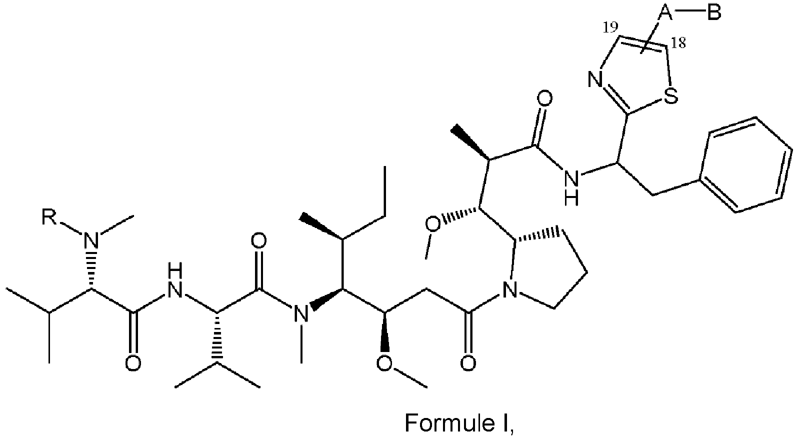 où :
A est un alkyle en C₁₋₆ linéaire ou ramifié attaché à un groupe thiazole en position 18 ou 19 ;
B est un groupe fonctionnel ; et
R est un alkyle ou H.

2. Analogue de la dolastatine 10 selon la revendication 1, où ledit alkyle en C₁₋₆ linéaire ou ramifié est attaché en position 18 sur ledit groupe thiazole.

3. Analogue de la dolastatine 10 selon l'une quelconque des revendications 1 à 2, où ledit groupe fonctionnel est un hydroxyle.

4. Analogue de la dolastatine 10 selon l'une quelconque des revendications 1 à 3, où R est un méthyle.

5. Analogue de la dolastatine 10 selon l'une quelconque des revendications 1 à 4, où A est un -(CH₂)- attaché en position 18, B est un -OH, et l'analogue de la dolastatine 10 a une structure : 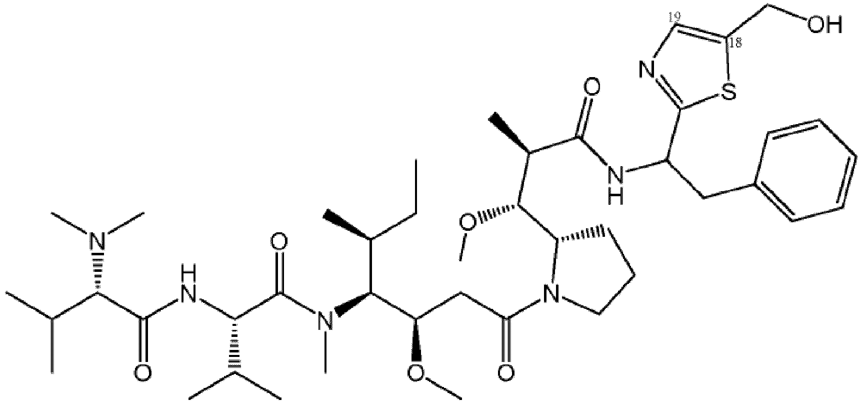 *(2S)-2-((S)-2-(diméthylamino)-3-méthylbutanamido)-N-((3R,4S,5S)-1-((2S)-2-((1R,* 2*R*)-3-((1-(5-(hydroxyméthyl)thiazol-2-yl)-2-phényléthyl)amino)-1-méthoxy-2-méthyl-3-oxopropyl)pyrrolidin-1-yl)-3-méthoxy-5-méthyl-1-oxoheptan-4-yl)-*N*,3-diméthylbutanamide.

6. Conjugué comprenant :
une fraction d'un analogue de la dolastatine 10 selon l'une quelconque des revendications 1 à 5,
une fraction fonctionnelle choisie dans le groupe constitué d'un agent bioactif, d'un agent de marquage et/ou d'un agent de diagnostic, et
une fraction de liaison reliant ladite fraction d'analogue de la dolastatine 10 et ladite fraction fonctionnelle, ladite fraction de liaison est éventuellement une liaison bioclivable ou un groupe bioclivable.

7. Conjugué selon la revendication 6, où ladite fraction de liaison comprend un disulfure.

8. Conjugué selon la revendication 6, où ladite fraction fonctionnelle est un analogue de la somatostatine.

9. Conjugué selon la revendication 8, où ledit analogue de la dolastatine 10 comprend un -CH₂-OH au niveau de son thiazole, ladite fraction fonctionnelle est une fraction d'octréotide, et ladite fraction de liaison comprend un disulfure.

10. Conjugué selon l'une quelconque des revendications 6 à 9, étant le conjugué 8b, le conjugué 8c ou le conjugué 8d : 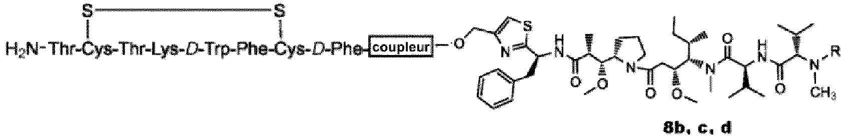
**8b** : Coupleur = -CO(CH₂)₂S-S(CH₂)₂NH- ; R=H
**8c** : Coupleur = -CO(CH₂)₂S-S(CH₂)₂NH- ; R=Me
**8d** : Coupleur = -COCH(NH₂)CH₂S-S(CH₂)₂NH- ; R=Me

11. Procédé de préparation de l'analogue de la dolastatine 10 selon la revendication 5, comprenant :
attacher un dérivé protégé par une amine de (2-(1-(λ²-azaneyl)-2-phényléthyl)thiazol-5-yl)méthanol **(Bu-5)** à une résine de support solide de chlorure de 2-chlorotrityle pour former ainsi un peptide naissant,
coupler un dérivé protégé par une amine de l'acide (2*R*,3*R*)-3-méthoxy-2-méthyl-3-((*S*)-pyrrolidin-2-yl) propanoïque **(Bu-1)** audit peptide naissant sur ladite résine,
coupler un dérivé protégé par une amine de l'acide (3*R*,4*S*,5*S*)-3-méthoxy-5-méthyl-4-(méthylamino) heptanoïque **(Bu-2)** audit peptide naissant sur ladite résine,
coupler un dérivé protégé par une amine de Val-OH audit peptide naissant sur ladite résine,
coupler le diméthyl-Val audit peptide naissant sur ladite résine, et
cliver l'analogue de la dolastatine 10 de ladite résine pour obtenir ainsi (2*S*)-2-((*S*)-2-(diméthylamino)-3-méthylbutanamido)-N-((3*R*,4*S*,5*S*)-1-((2*S*)-2-((1 *R*,2*R*)-3-((1-(5-(hydroxyméthyl)thiazol-2-yl)-2-phényléthyl)amino)-1-méthoxy-2-méthyl-3-oxopropyl)pyrrolidin-1-yl)-3-méthoxy-5-méthyl-1-oxoheptan-4-yl)-N,3-diméthylbutanamide.

12. Procédé de préparation du conjugué selon l'une quelconque des revendications 6 à 10, comprenant :
former ladite fraction fonctionnelle sur une résine de support solide en utilisant des protocoles de synthèse de peptides à l'état solide,
où ladite fraction fonctionnelle comprend un groupe réticulable,
former ledit analogue de la dolastatine 10 en commençant à partir dudit groupe réticulable, formant ainsi ladite fraction de liaison sur une résine de support solide en utilisant des protocoles de synthèse de peptides à l'état solide, et
libérer le conjugué de ladite résine.

13. Composition pharmaceutique comprenant l'analogue de la dolastatine 10 selon l'une quelconque des revendications 1 à 5, et un support pharmaceutiquement acceptable.

14. Composition pharmaceutique comprenant le conjugué selon l'une quelconque des revendications 6 à 10, et un support pharmaceutiquement acceptable.
